# EUROPEAN PATENT SPECIFICATION

(11) **EP 3 551 214 B1**
(45) Date of publication and mention of the grant of the patent: **13.03.2024**
(21) Application number: 17878004.5
(22) Date of filing: 06.12.2017
(51) Int. Cl.: A61K 38/19, A61K 38/22, A61K 39/395, A61P 3/04, A61P 1/14, A61P 3/10

(54) **TREATMENT OF OBESITY AND EATING DISORDERS**
BEHANDLUNG VON FETTLEIBIGKEIT UND ESSSTÖRUNGEN
TRAITEMENT DE L'OBÉSITÉ ET DES TROUBLES ALIMENTAIRES

(30) Priority: 06.12.2016 AU 2016905018
(43) Date of publication of application: 16.10.2019
(73) Proprietor: St Vincent's Hospital Sydney Limited, Darlinghurst, NSW 2010 (AU)
(72) Inventor: BREIT, Samuel Norbert, Gordon, New South Wales 2072 (AU)
(74) Representative: D Young & Co LLP
(86) International application number: PCT/AU2017/000262
(87) International publication number: WO 2018/102854

(56) References cited:
- WO-A1-97/46249
- WO-A1-2005/099746
- WO-A1-2009/021293
- WO-A1-2009/046495
- WO-A1-2013/113008
- WO-A1-2014/100689
- WO-A1-2015/144855
- WO-A1-2015/197446
- WO-A1-2015/198199
- WO-A1-2016/049470
- WO-A1-2016/069921
- WO-A2-96/34885
- WO-A2-2009/108340
- WO-A2-2012/138919
- BRYSON J M: "The future of leptin and leptin analogues in the treatment of obesity", DIABETES, OBESITY AND METABOLISM, BLACKWELL SCIENCE, vol. 2, 1 January 2000 (2000-01-01), pages 83-89, XP002219935, ISSN: 1462-8902, DOI: 10.1046/J.1463-1326.2000.00052.X
- ARZOLA-PANIAGUA M.A. ET AL.: 'Efficacy of an Orlistat-Resveratrol Combination for Weight Loss in Subjects with Obesity: A Randomized Controlled Trial' OBESITY vol. 24, no. 7, July 2016, pages 1454 - 63, XP055491908
- CHRYSOVERGIS K. ET AL.: 'NAG-l/GDF-15 prevents obesity by increasing thermogenesis, lipolysis and oxidative metabolism' INTERNATIONAL JOURNAL OF OBESITY vol. 38, no. 12, 2014, pages 1555 - 64, XP055489406
- TSAI V.W-W ET AL.: 'TGF-b Superfamily Cytokine MIC-1/GDF15 Is a Physiological Appetite and Body Weight Regulator' PLOS ONE, [Online] vol. 8, no. 2, 28 February 2013, XP055491912 Retrieved from the Internet: <URL:https://www.ncbi.nlm.nih.gov/pmc/artic les/PMC3 5 85300/pdf/pone.00551 74.pdf> [retrieved on 2018-02-23]
- MACIA L. ET AL.: 'Macrophage Inhibitory Cytokine 1 (MIC-1/GDF15) Decreases Food Intake, Body Weight and Improves Glucose Tolerance in Mice on Normal & Obesogenic Diets' PLOS ONE, [Online] vol. 7, no. 4, 2012, page E34868, XP002687296 Retrieved from the Internet: <URL:https://www.ncbi.nlm.nih.gov/pmc/artic les/PMC3 3 25 923/pdf/pone. 0034868. pdf> [retrieved on 2018-02-23]
- BREIT S.N. ET AL.: 'The TGF-P superfamily cytokine, MIC-1/GDF15: A pleotropic cytokine with roles in inflammation, cancer and metabolism' GROWTH FACTORS, OCT vol. 29, no. 5, 2011, pages 187 - 195, XP008152690
- DEPAOLI A.M. ET AL.: 'Leptin in common obesity and associated disorders of metabolism' JOURNAL OF ENDOCRINOLOGY vol. 223, no. 1, October 2014, pages T71 - 8 1, XP055491914
- PAZ-FILHO G. ET AL.: 'Leptin treatment: Facts and expectations' METABOLISM vol. 64, no. 1, January 2015, pages 146 - 56, XP029112083
- BANKS W.A. ET AL.: 'Principles of strategic drug delivery to the brain (SDDB): Development of anorectic and orexigenic analogs of leptin' PHYSIOLOGY & BEHAVIOUR vol. 105, no. 1, 2011, pages 145 - 149, XP028305170
- MAK R.H. ET AL.: 'Exploiting the therapeutic potential of leptin signalling in cachexia' CURR OPIN SUPPORT PALLIAT CARE vol. 8, no. 4, December 2014, pages 352 - 357, XP055436046

## Description

### TECHNICAL FIELD

The present disclosure relates to a composition comprising a first agent comprising MIC-1 and/or an active MIC-1 fragment; and a second agent comprising leptin and/or an active leptin fragment. The composition may be for use in methods of controlling body weight and/or appetite and for use in methods for the treatment or prevention of Type 2 diabetes (T2D) and/or one or more complications of T2D, obesity or being overweight.

### BACKGROUND

The control of body weight is a complex process that is multifactorial and influenced by a number of factors including appetite, food ingestion and excretion, energy utilisation and expenditure. A number of soluble mediators are known to be involved in regulating various aspects of this process and include systemic- and/or nervous system-derived hormones, cytokines and other mediators such as ghrelin, glucagon-like peptide 1 (GLP-1), amylin, insulin, cholecystokinin (CCK), proopiomelanocortin (POMC), agouti-related peptide (AgRP), and neuropeptide Y (NPY). Normal weight control is important to good health and overweight, and especially obesity, may greatly increase morbidity and mortality in individuals. On the other hand, having a lower than average weight can also be problematic, and in developed societies, where sufficient food is available, this is more frequently due to diseases including some chronic inflammatory disorders, chronic renal and cardiac failure, liver cirrhosis, chronic obstructive pulmonary disease (COPD), eating disorders such as anorexia nervosa, and cancer. Especially in the late stages of cancer, anorexia/cachexia is common (occurring in most terminally ill cancer patients), and is responsible for about a quarter of all cancer-related deaths.

Some years ago, the present Applicant cloned and characterised a novel human TGF-b superfamily cytokine that was named macrophage inhibitory cytokine-1 (MIC-1)¹⁻⁷, but has since also become known, amongst others, as prostate derived factor (PDF), placental bone morphogenetic protein (PLAB), and growth/differentiation factor-15 (GDF-15)⁷. Under resting conditions, placenta is the only tissue expressing large amounts of MIC-1⁷, but epithelial cells in a wide variety of other organs also normally express smaller amounts of MIC-1 mRNA such that MIC-1 circulates in all individuals with a broad normal range of 0.15-1. 15 ng/ml serums. These serum levels increase with age and a number of physiological variants such as body mass index (BMI), but are also increased in many different disease processes, especially those associated with malignancy, inflammation and injury^{7, 9-12}. In some of these, especially malignancies, such as those of the breast, prostate, pancreas, ovary and colon^{10-12, 14}, these serum levels can rise dramatically. In addition, in a study of several hundred patients with colonic polyps or colon cancer¹⁵, the present Applicant showed that elevation of serum levels of MIC-1 occurs in a progressive stepwise manner, reflecting colon cancer pathogenesis, with progression from normal to benign and then to dysplastic colonic polyps and finally colon cancer. However, whilst MIC-1 serum levels increase in disease such as these cancers, its overall biological effect, in most instances, is likely to be beneficial, as animal studies have indicated that MIC-1 over-expression mitigates disease¹⁶⁻¹⁹. Most importantly, a recent study demonstrated that transgenic mice over-expressing MIC-1, whether on a chow or high fat diet (HFD), have a markedly prolonged life expectancy²⁰. This suggests that disease associated elevation of serum MIC-1 levels may represent an incompletely effective attempt to control the pathological process in question.

The present Applicant has previously found that mice with tumour xenografts, engineered to over-express human MIC-1, became anorexic/cachectic^{21,22}. Further, it was observed that the serum levels of the tumour-derived MIC-1 in the mice were proportional to the degree of weight loss, with more marked effects occurring in mice with serum levels greater than about 8-10 ng/ml. Importantly, these serum levels are well within the range of that commonly seen in patients with diseases such as advanced cancer (ie cancer, which has typically metastasized, and which is not considered curable but responds to treatment (eg by disease-directed therapy) to prolong life). Moreover, mice with tumours over-expressing MIC-1 were found to eat less and lose a substantial amount of fat and muscle, which could be reversed, without modifying tumour progression, by administering an antagonistic anti-MIC-1 monoclonal antibody^{21,22}. In addition, in a comparative study, it was found that the administration of recombinant MIC-1 to mice reproduced these findings²¹ and were consistent with studies of humans correlating serum MIC-1 levels with reduced BMI or anorexia/cachexia syndromes^{21, 23-25}. Also, the studies with MIC-1 over-expressing transgenic mice found that these mice were lean, protected from the development of obesity and showed an improved level of glucose tolerance when placed on an obesogenic diet^{21, 26, 27}. It was also considered that these transgenic mice may have increased thermogenesis^{20, 28}. Further, it has been found that mice bearing a germline gene deletion of *MIC-1* are obese, eat more and weigh more throughout their life than syngeneic mice. These findings suggest that MIC-1 plays a role in the physiological regulation of energy homeostasis and that this pathway becomes subverted in disease states leading to an anorexia/cachexia syndrome.

As mentioned above, mice with MIC-1 over-expressing tumours become anorexic/cachectic and have a decreased food intake. This is mediated by actions on the hypothalamus and brainstem, which is a major mechanism for the observed weight loss²¹. This is supported by the finding that surgical lesioning of the brainstem area postrema (AP) and the medial (m) nucleus of solitary tract (NTS) of mice prevents the anorexic actions of recombinant MIC-1²⁹. However, it is suspected that there is much more involved and, notably, the studies described above did not exclude metabolic actions of MIC-1 outside of the central nervous system (CNS) or its possible actions on different CNS centres. Moreover, the present Applicant considers that the metabolic actions of MIC-1 may interact with other agents involved in appetite control and/or energy homeostasis (eg some adipokines and peptide hormones such as ghrelin and proopiomelanocortin). This led to the investigation of whether there is interaction between the effects of MIC-1 and the adipokine, leptin.
WO 2005/099746 teaches a method of modulating appetite and/or body weight in a subject, said method comprising administering to said subject an effective amount of a MIC-1-modulating agent.

### SUMMARY

The present invention provides a composition comprising:
(i) a first agent comprising MIC-1 and/or an active MIC-1 fragment; and
(ii) a second agent comprising leptin and/or an active leptin fragment;
and optionally containing a pharmacologically acceptable carrier and/or excipient..

Preferably, the first agent comprises MIC-1.

The present invention also provides a composition according to the invention for use in a method of controlling body weight and/or appetite, wherein said composition is administered to a subject selected from a subject that is overweight or obese, a subject with diet-induced obesity (DIO), a subject suffering from Type 2 diabetes (T2D), a subject suffering from non-alcoholic steatohepatitis (NASH), and a subject showing insulin resistance.

The present invention also provides a composition according to the invention for use in a method for the treatment or prevention of Type 2 diabetes (T2D) and/or one or more complications of T2D, obesity or being overweight.

The subject may be an overweight or obese subject and, in one embodiment, the subject may be an overweight or obese subject with a low serum leptin level.

The composition may be suitable for, for example, oral, buccal, nasal, intramuscular and intravenous administration.

The present invention also provides a kit comprising first and second containers (eg vials), wherein the first container contains a first agent comprising MIC-1 and/or an active MIC-1 fragment, and the second container contains a second agent comprising leptin and/or
an active leptin fragment; optionally packaged with instructions for the use according to the invention.

### BRIEF DESCRIPTION OF FIGURES

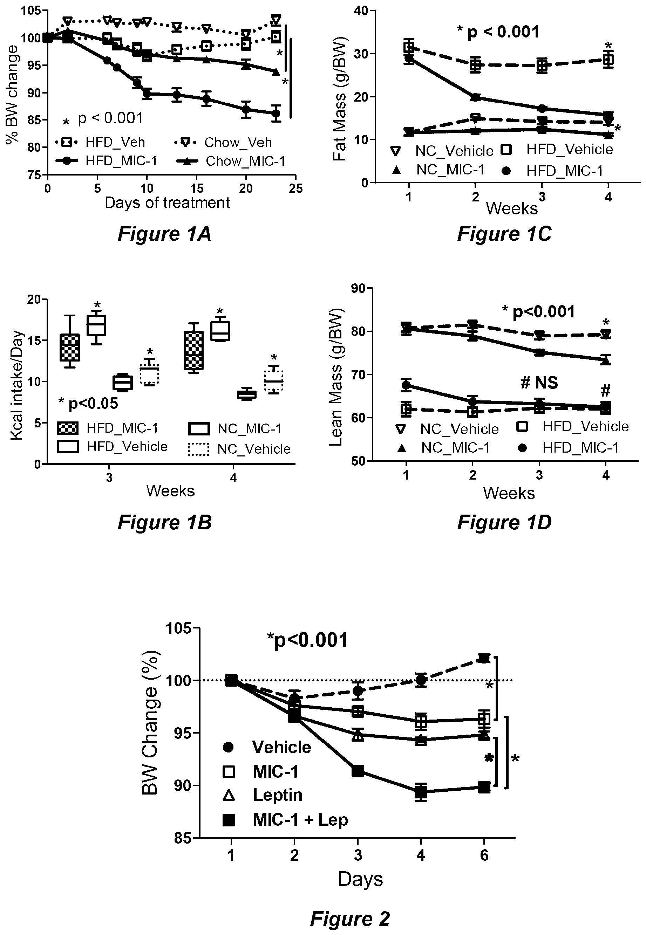

**Figure 1** provides graphical results obtained from a study described in Example 1 hereinafter: (A) shows the percentage change in body weight (% BW change) of CHOW fed mice (Chow) and or mice with diet-induced obesity (DIO) (denoted in the figure as HFD) administered with agents as shown on the graph consisting of vehicle (Veh) or MIC-1 via an osmotic minipump; (B) shows food intake (Kcal intake/Day) by the same mice after 3 and 4 weeks; and (C) and (D) show the amount of fat mass (g/BW) and lean mass (g/BW) of the same mice respectively;
**Figure 2** provides graphical results obtained from a study described in Example 2 hereinafter: the graph showing the percentage change in body weight (% BW change) of normal chow fed mice infused (using a standard osmotic minipump) with agents as shown on the graph consisting of either vehicle (●), recombinant MIC-1 (□), leptin (A), or MIC-1 plus leptin (■) for six (6) days;

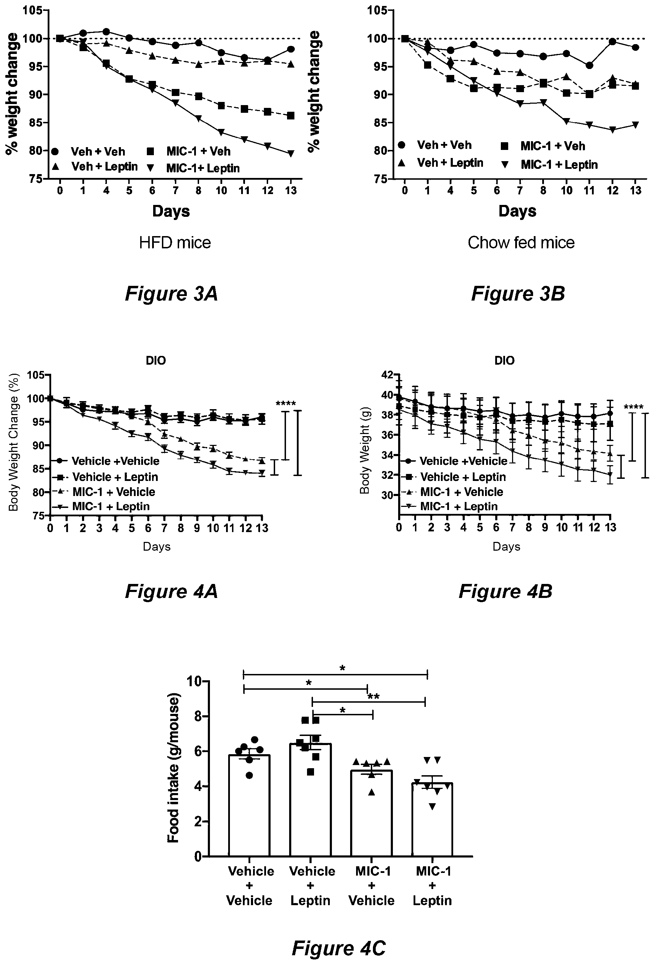

**Figure 3** provides graphical results obtained from a study described in Example 3 hereinafter: graph showing the percentage change in body weight (% weight change) of either (A) HFD mice or (B) normal chow fed mice infused with binary combinations of agents separately in two osmotic minipumps as shown on the graph consisting of vehicle (veh) and vehicle (●), recombinant MIC-1 and vehicle (■), leptin and vehicle (▲), and MIC-1 plus leptin (▼);
**Figure 4** provides graphical results obtained from a study described in Example 4 hereinafter: (A) shows the percentage change in body weight (% BW change) and (B) shows the body weight amount (g) of DIO mice infused with agents as shown on the graph with vehicle + vehicle (●), vehicle + recombinant leptin (■), recombinant MIC-1 + vehicle (A), or recombinant MIC-1 + recombinant leptin (▼); while (C) compares the level of food intake by the mice measured as 3-day accumulated food consumption as g/mouse;

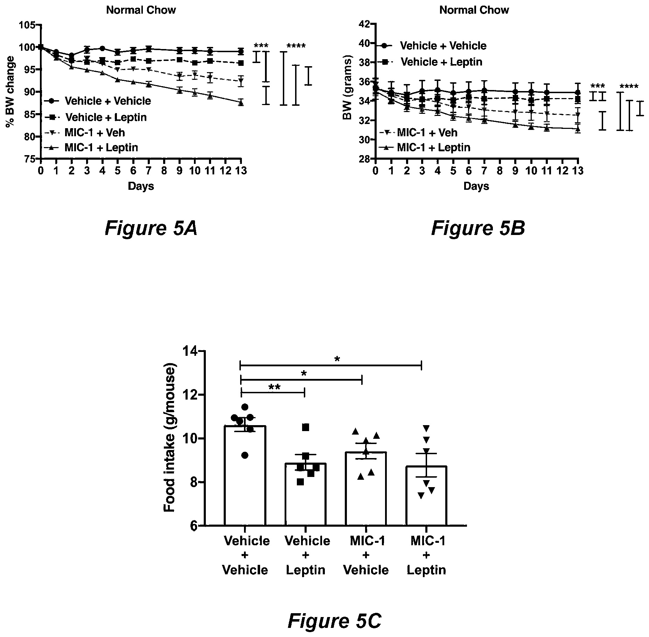

**Figure 5** provides graphical results obtained from the Example 4 study: (A) shows the percentage change in body weight (% BW change) and (B) shows the body weight amount (g) of normal CHOW fed mice infused with agents as shown on the graph consisting of two osmotic minipumps with vehicle + vehicle (●), vehicle + leptin (■), MIC-1 + vehicle (▲), or MIC-1 + leptin (▼); while (C) compares the level of food intake by the mice measured as 3-day accumulated food consumption as g/mouse;

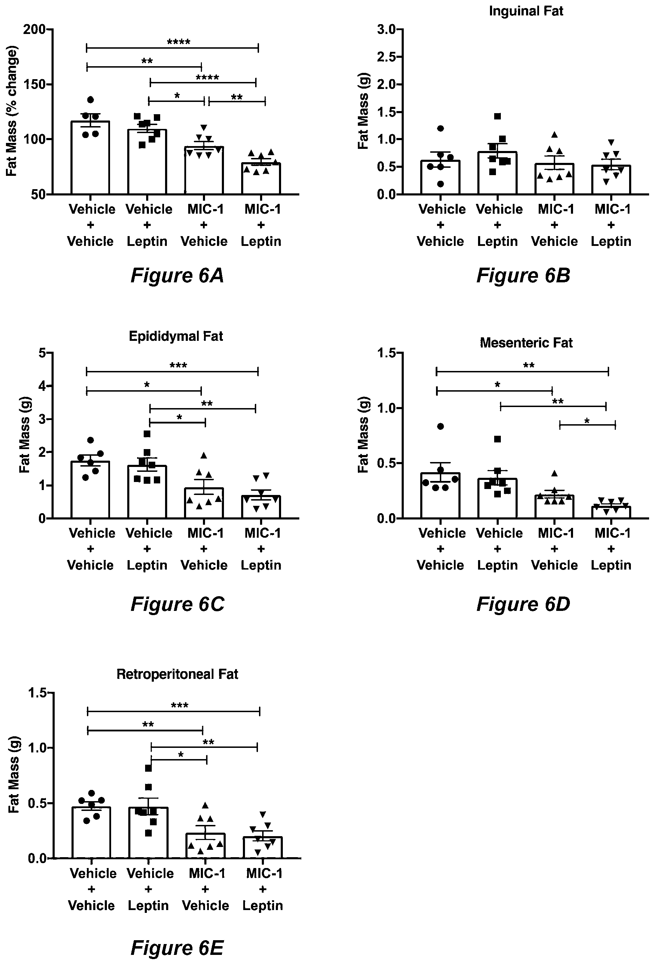

**Figure 6** provides graphical results obtained from a study described in Example 4 showing that the combination of MIC-1 and leptin treatments induced greater fat mass reduction in DIO mice than either agent alone: (A) Precent change in fat mass, quantified by DEXA, between that determined immediately prior to the experiment and at the end of the experiment was calculated; (B) Inguinal fat mass, (C) Epididymal fat mass, (D) Mesenteric fat mass and (E) Retroperitoneal fat masses were quantified upon dissection at the conclusion of the experiment;

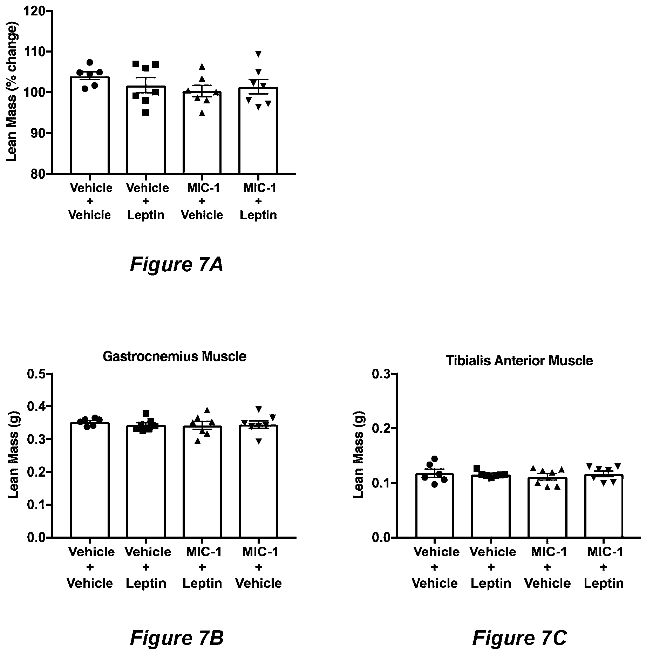

**Figure 7** provides graphical results obtained from a study described in Example 4 showing that MIC-1 and leptin, singly or in combination, had no effect on lean mass of DIO mice: (A) Percent change of in lean mass, quantified by DEXA, between that determined immediately prior to the experiment and at the end of the experiment was calculated; (B) Gastrocnemius muscle mass and (C) Tibialis anterior muscle mass were quantified upon dissection at the conclusion of the experiment;

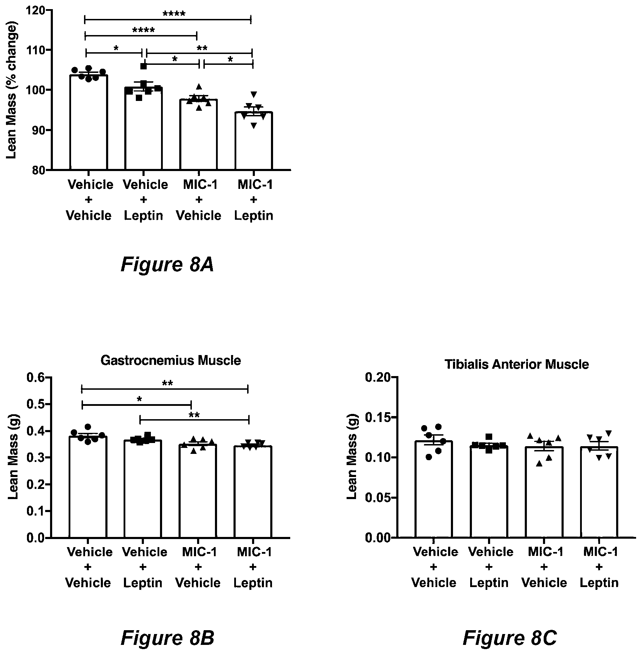

**Figure 8** shows the results of experimentation described in Example 4. The combination of MIC-1 + leptin treatment of normal CHOW fed mice induced a greater reduction in lean mass compared to either of these alone: (A) Percent change in lean mass, quantified by DEXA, between that determined immediately prior to the experiment and at the end of the experiment was calculated; (B) Gastrocnemius muscle mass and (C) Tibialis anterior muscle mass were quantified after dissection at the end of the experiment;

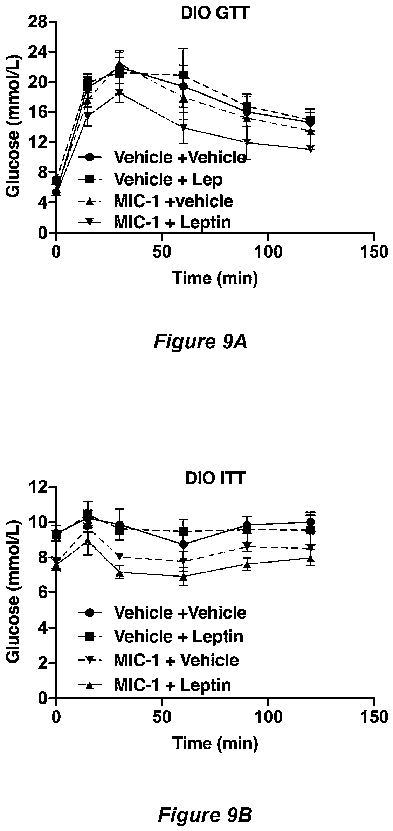

**Figure 9** provides the results of (A) intraperitoneal glucose tolerance test (GTT) and (B) insulin sensitivity test (ITT) performed on DIO mice at day 14 of the experiments described in Example 4 showing that MIC-1 + leptin results in improved GTT and ITT compared to either leptin or MIC-1 alone. DIO mice were infused with agents as shown on the graph consisting of two osmotic minipumps with vehicle + vehicle (●), vehicle + recombinant leptin (■), recombinant MIC-1 + vehicle (▲), or recombinant MIC-1 + recombinant leptin (▼);

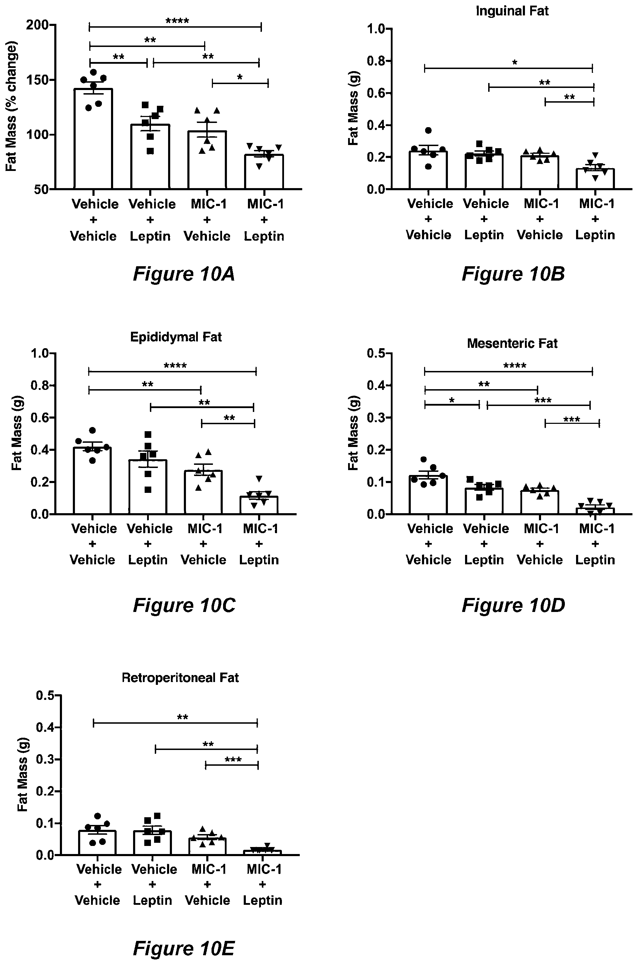

**Figure 10** provides graphical results obtained from a study described in Example 4 showing that the combination of MIC-1 and leptin treatments induced greater fat mass reduction in normal CHOW fed mice than either agent alone: DIO mice were infused with agents as shown on the graph consisting of two osmotic minipumps with vehicle + vehicle (●), vehicle + recombinant leptin (■), recombinant MIC-1 + vehicle (▲), or recombinant MIC-1 + recombinant leptin (▼). (A) Precent change in fat mass, quantified by DEXA, between that determined immediately prior to the experiment and at the end of the experiment was calculated; (B) Inguinal fat mass, (C) Epididymal fat mass, (D) Mesenteric fat mass and (E) Retroperitoneal fat mass were quantified upon dissection at the conclusion of the experiment;

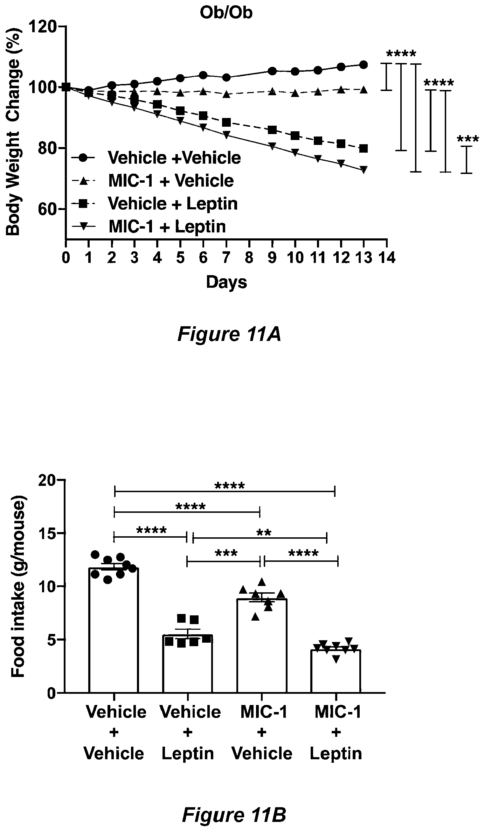

**Figure 11** provides graphical results obtained from the experimentation described in Example 5. It was found that the combination of MIC-1 + leptin induced greater body weight loss and food intake reduction in ob/ob mice than either agent alone. Male ob/ob mice fed with normal-chow for 10 weeks, were treated using two osmotic pumps in binary combinations of MIC-1 (0.5 µg/gBW/day) + vehicle, leptin (0.25 µg/gBW/day) + vehicle, MIC-1 + leptin or vehicle + vehicle for 19 days (A) Body weight was monitored periodically and is presented as percentage change compared to that determined immediately prior to the start of the experiment; and (B) Food intake in was measured based on 3-day accumulated food consumption as g/mouse;

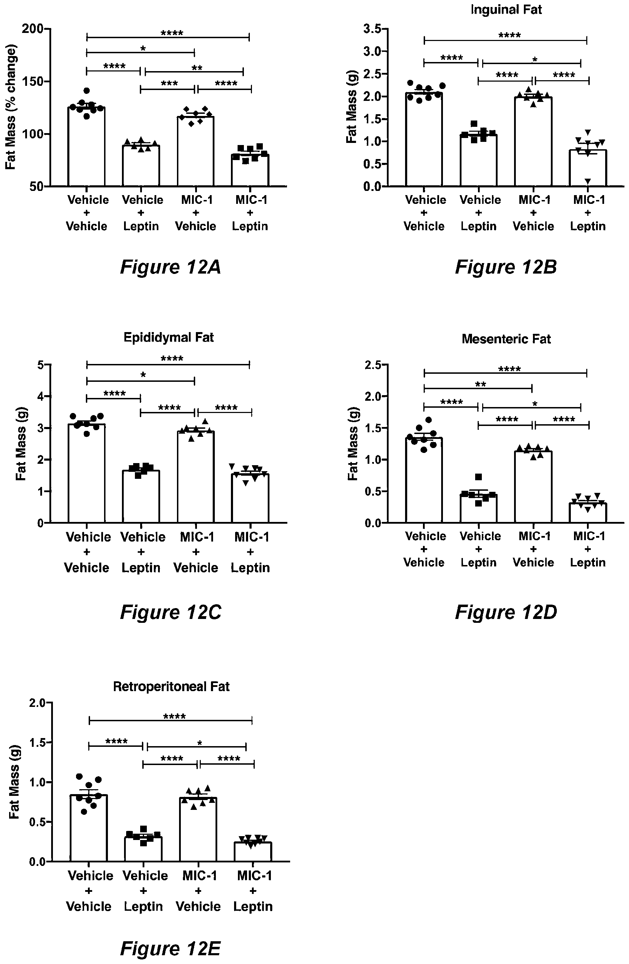

**Figure 12** shows the results of the treatment of combinations of vehicle, MIC-1 and leptin on fat mass reduction in ob/ob mice. Mice were treated using two osmotic pumps in binary combinations of MIC-1 (0.5 µg/gBW/day) + vehicle, leptin (0.25 µg/gBW/day) + vehicle, MIC-1 + leptin or vehicle + vehicle for 19 days: (A) Percent change in fat mass, quantified by DEXA, between that determined immediately prior to the experiment and at the end of the experiment was calculated; (B) Inguinal fat mass, (C) Epididymal fat mass, (D) Mesenteric fat mass and (E) Retroperitoneal fat mass were quantified after dissection at the end of the treatment period;

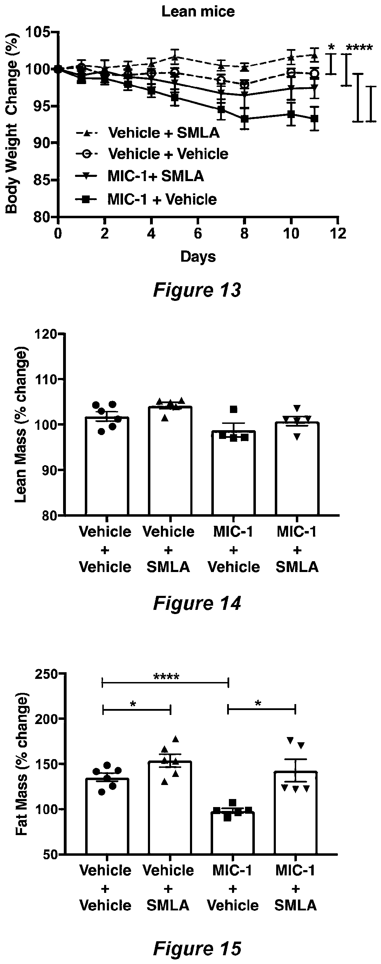

**Figure 13** shows the results of treatments of mice with a leptin antagonist (SMLA). The leptin antagonist reversed MIC-1-induced body weight reduction: Male mice fed on a normal chow diet for 30 weeks were infused using two osmotic pumps in binary combinations of MIC-1 (0.5 µg/gBW/day) + vehicle, SMLA (1.5 µg/gBW/day) + vehicle, MIC-1 + SMLA or vehicle + vehicle for 14 days. Body weight was monitored daily over the first 11 days of the treatment regimen and is presented as percentage change compared to that determined immediately prior to the start of the experiment;
**Figure 14** provides graphical results of a study investigating the effect of MIC-1 and a leptin antagonist on modulating muscle mass. Male C57BL/6J mice fed with normal chow for 30 weeks were treated using two osmotic pumps in binary combinations of MIC-1 (0.5 µg/gBW/day) + vehicle, SMLA (1.5 µg/gBW/day) + vehicle, MIC-1 + SMLA or vehicle + vehicle for 14 days. The percent change of in lean mass, quantified by DEXA, between that determined immediately prior to the experiment and at day 11 was calculated; and
**Figure 15** provides graphical results showing that treatments with a leptin antagonist inhibited the effect of MIC-1-induced fat mass reduction: Mice fed with normal chow for 30 weeks were treated using two osmotic pumps in binary combinations of MIC-1 (0.5 µg/gBW/day) + vehicle, SMLA (1.5 µg/gBW/day) + vehicle, MIC-1 + SMLA or vehicle + vehicle for 14 days. Percent change in fat mass, quantified by DEXA, between that determined immediately prior to the experiment and at the end of the experiment at day was calculated.

### DETAILED DESCRIPTION

An investigation into a possible interaction between MIC-1 and the adipokine, leptin, using an obese mouse model, determined that treatment with MIC-1 and leptin caused significantly greater weight loss than that observed with MIC-1 or leptin alone (see Examples hereinafter).

The composition according to the invention may be for use in a method of controlling body weight (eg achieving weight loss) and/or appetite in a subject wherein said composition is administered to a subject selected from a subject that is overweight or obese, a subject with diet-induced obesity (DIO), a subject suffering from Type 2 diabetes (T2D), a subject suffering from non-alcoholic steatohepatitis (NASH), and a subject showing insulin resistance.

The composition according to the invention may be used to achieve appetite control and/or body weight control in a subject. For example, for body weight control, the composition according to the invention may achieve weight loss in a subject or, at least, to prevent further gain of body weight in a subject. The subject may
be a subject in need of body weight control such as, for example, an overweight or obese subject (for example, a subject with diet-induced obesity (DIO)) and/or a reduction in appetite. For the purposes of the present disclosure, an overweight subject is considered to be a subject with a body mass index (BMI) of 25 to 30, and an obese subject is considered to be a subject with a BMI of 30 or more^{33, 61}.

Preferably, the composition for use according to the invention may be for use in a method of decreasing body weight (ie achieving weight loss) or preventing body weight gain.

The subject may be an overweight or obese subject with a lower than normal serum leptin level (ie a serum leptin level that is lower than the typical range for a normal subject) or, otherwise, a relatively low serum leptin level for an obese subject or relatively low serum level given the degree of adiposity present in the subject.

For normal (ie non-obese) human subjects, the normal serum level range of leptin is broad; for example, about 3.5 to 14.0 ng/ml for adult women and 4.5 to 25.5 ng/ml for adult men³¹ after overnight fasting. A lower than normal serum leptin level may be regarded as a level at the lower end of these ranges (eg less than about 6 ng/ml for adult women and less than about 12 ng/ml for adult men) or lower than these ranges.

For most obese human subjects, their serum leptin level (following overnight fasting) will be high (nb. it has been found that obese humans have (on average) leptin levels four times higher than non-obese individuals⁶⁰) and correlates with BMI⁶⁸. For example, it has been reported that the mean serum leptin level after overnight fasting for obese subjects is 31.3 ng/ml⁶⁰ (nb. in this study, obese subjects were considered as having a BMI of ≥ 27.3 for men and ≥ 27.8 for women). Elsewhere, it has been reported that the mean serum leptin level for obese adult women is 38.2 and that the mean serum leptin level for obese adult men is 27.0⁶¹. Given such knowledge, those skilled in the art can readily determine serum leptins levels which would be regarded as being relatively low for an obese subject. However, in one embodiment, a relatively low serum leptin level for an obese subject (after overnight fasting) may be, for an adult woman, less than about 30 ng/ml or, more preferably, less than 25 or less than 20 ng/ml, and for an adult man, less than about 20 ng/ml or, more preferably, less than 15 ng/ml.

Population studies have suggested that about 10% of obese individuals have low serum leptin levels⁵⁹. Lower than normal serum leptin levels or levels which are relatively low for an obese subject may be caused by, for example, impairment of leptin regulation (especially, reduced leptin production by adipose tissue⁶⁷) which may or may not be associated with certain diseases and conditions such as congenital leptin deficiency³⁴, intra-uterine growth retardation (IUGR)³² and other factors. Subjects with a lower than normal serum leptin level or relatively low serum leptin level (for an obese subject) can be identified by measuring serum leptin levels in accordance with any suitable assay method known to those skilled in the art including standard immunoassays (eg a leptin ELISA). Thus, in a particular embodiment, the composition for use according to the invention may be for use in a method of decreasing body weight (ie achieving weight loss) or preventing body weight gain in an overweight or obese subject with a lower than normal serum leptin level or a relatively low serum leptin level for an obese subject. It is considered that in accordance with the results achieved in the investigation described hereinafter, administering to such a subject an agent comprising, for example, MIC-1 and/or an active MIC-1 fragment *and* an agent comprising leptin and/or an active leptin fragment will be more effective for body weight control than the use of MIC-1 and/or an active MIC-1 fragment alone.

In the kit according to the invention, the first agent comprising MIC-1 and/or an active MIC-1 fragment may, for example, be administered to the subject concurrently (eg in combination) with the second agent comprising leptin and/or an active leptin fragment, or otherwise the first agent comprising MIC-1 and/or an active MIC-1 fragment and the second agent comprising leptin and/or an active leptin fragment may be administered to the subject consecutively and in either order. As used herein, it is to be understood that when administered consecutively, the respective agents may be administered one after another with practically no time interval (ie one is administered effectively immediately after the other) or, otherwise, after an interval of 1 to 5 minutes or more (eg 10 minutes, 30 minutes, 60 minutes, 4 hours or 12 hours). Where the first and second agents are administered consecutively, they may each be formulated for administration in a pharmaceutical composition form including a pharmacologically acceptable carrier and/or excipient, which may be the same or different. The pharmaceutical compositions may optionally comprise other substances such as absorption enhancers including surfactants (eg sodium lauryl sulphate, laureth-9, sodium dodecylsulphate, sodium taurodihydrofusidate and poly oxyethylene ethers) and chelating agents (eg EDTA, citric acid and salicylates). The pharmaceutical compositions may be suitable for, for example, oral, buccal, nasal, intramuscular and intravenous administration. In some embodiments, the pharmaceutical compositions are particularly suitable for nasal administration (ie intranasal delivery), which has been previously shown as an efficacious route for the administration of TGF-β superfamily members (GDF-5 and GDNF) and other proteins and peptides (recently reviewed by Amirah E-E A and BL Waszczak⁵⁶). Suitable pharmaceutical compositions for nasal administration include, but are not limited to, liposomal compositions⁵⁷ and lipid microemulsions⁵⁸.

Typically, the pharmaceutical compositions will be administered to the subject in an amount which is effective for controlling body weight and/or appetite. The first agent may be administered to provide, for example, an amount of the agent comprising MIC-1 and/or an active MIC-1 fragment that is between about 0.01 and about 100 mg/kg body weight per day, or between about 0.05 and 25 mg/kg body weight per day of the agent comprising MIC-1
and/or an active MIC-1 fragment. The second agent may be administered to provide, for example, an amount of the agent comprising leptin and/or an active leptin fragment that is between about 0.01 and about 5000 mg/kg body weight per day, between about 10 and 1000 mg/kg body weight per day of the agent comprising leptin and/or an active leptin fragment, or between about 100 and 500 mg/kg body weight per day of the agent comprising leptin and/or an active leptin fragment. The pharmaceutical compositions may be intended for single daily administration, multiple daily administration, or controlled or sustained release, as needed to achieve the most effective results.

In the composition according to the invention, the first agent comprising MIC-1 and/or an active MIC-1 fragment and the second agent comprising leptin and/or an active leptin fragment are formulated in combination for administration in a single composition. The combination composition may further comprise, for example, a pharmacologically acceptable carrier and/or excipient, optionally together with others substances such as absorption enhancers (including surfactants and chelating agents) and protease inhibitors (such as aprotinin (trypsin/chymotrypsin inhibitor), amastatin, bestatin, boroleucine, and puromycin (aminopeptidase inhibitors)) for improving bioavailability, especially oral bioavailability. Such a combination pharmaceutical composition may be suitable for, for example, oral, buccal, nasal, intramuscular and intravenous administration. In some embodiments, the pharmaceutical composition is particularly suitable for nasal administration (ie intranasal delivery).

Typically, a combination pharmaceutical composition will be administered to the subject in an amount which is effective for controlling body weight and/or appetite. Thus, the combination composition may be administered to provide, for example, an amount of the agent comprising MIC-1 and/or an active MIC-1 fragment that is between about 0.01 and about 100 mg/kg body weight per day or between about 0.05 and 25 mg/kg body weight per day, and an amount of the agent comprising leptin and/or an active leptin fragment that is between about 0.01 and about 5000 mg/kg body weight per day, between about 100 and 1000 mg/kg body weight per day, or between about 100 and 500 mg/kg body weight per day of the agent comprising leptin and/or an active leptin fragment. The combination pharmaceutical composition may be intended for single daily administration, multiple daily administration, or controlled or sustained release, as needed to achieve the most effective results. However, notwithstanding the above, it will be understood by those skilled in the art that the administered amount of the combination composition, and the frequency of administration for any particular subject, may vary and depend upon a variety of factors including the activity of the active agents (eg the first and second agents), the metabolic stability and length of action of the active agents, the age, body weight, sex, mode and time of administration, and the rate of excretion of the active agents.

The composition according to the invention may involve a first agent that comprises a recombinant
MIC-1 or synthetic MIC-1 (ie MIC-1 produced by protein synthesis techniques) comprising, preferably, a dimer of a polypeptide comprising a native MIC-1 amino acid sequence (eg the native amino acid sequence of a mature (processed) MIC-1 protein) or a variant MIC-1 amino acid sequence. Suitable variants of MIC-1 amino acid sequences may include a naturally occurring or non-natural variant amino acid sequence of a native amino acid sequence that may include one or more minor sequence variations which, preferably, do not substantially alter the function of the polypeptide (eg despite the variation(s), the polypeptide maintains the ability of binding to and activating the MIC-1 receptor, known as the GDNF family receptor α-like GFRAL)^{30, 69, 70}). The native human MIC-1 amino acid sequence was originally published by the present Applicant in International patent publication No WO 97/000958 (wherein MIC-1 is referred to as CL13). An example of a naturally occurring variant of that sequence, which is suitable for use in the method of the first aspect, comprises a His→Asp substitution at position 6 (ie H6D) of the mature MIC-1 amino acid sequence. Other variants of MIC-1 amino acid sequences may include one or more conservative amino acid substitutions such as: G, A, V, I, L, M; D, E; N, Q; S, T; K, R, H; F, Y, W, H; and P, Nα-alkylamino acids. Other substitutions may include the substitution of one or more L-amino acid(s) with a D-amino acid(s). Preferably, any amino acid substitution comprises a substitution with an amino acid selected from the twenty (20) standard amino acids encoded by the genetic code (ie the canonical amino acids). Some suitable examples of amino acid substitutions have been described in International patent publication no WO 2017/202936 such as N3E, P11E, H18E, R21E, A30E, A47E, R53E, A54E, M57E, M57L, R67E, L68E, A75E, A81E, P85E, M86L, L105E and K107E, which may improve solubility, improve stability and/or prevent oxidation of the MIC-1. However, amino acid substitutions with non-canonical amino acids such as, for example, certain Nα-alkylamino acids (eg N-methyl glycine (sarcosine) and N-methyl alanine), and other amino acids such as 2-aminobutyric acid (Abu), naphthylalanine (Nal), amino isobutyric acid, 3-aminoadipic acid (Aad), ornithine, citruline, amino-oxyserine, homo-arginine, norleucine (Nle), aminosuberic acid and β-2- and β-3-napthylalanine, ring-substituted phenylalanine (Phe) derivatives (eg 2,3,4,5,6-pentafluoro-phenylalanine, 4-chloro-phenylalanine, methyl-phenylalanine and phosphono-phenylalanine), phospho-tyrosine (pTyr), selenocysteine and selenomethionine, are also contemplated. Other sequence variations that may be present include one or more amino acid deletion (such as, for example, the deletion of Asn and position 3 (des-N3)) or addition (eg insertion). Other additions that may be made to, for example, the N- or C-terminal sequence may comprise the addition of a single amino acid (eg Ala), short amino acid sequences (eg 2 to 10 amino acids in length) or long amino acid sequences (eg 11 or more amino acids) which may confer various additional functionalities or properties, such as improved bioavailability, extended circulating half life, improved solubility and/or stability, and improved protein recovery or expression (eg a fusion partner). Numerous examples of MIC-1 agents comprising an additional amino acid sequence (of 3 to 36 amino acids in length or more) at the N-terminal (ie an N-terminal extension)
have been described in WO 2017/202936 and are considered as suitable for use as described herein. MIC-1 agents comprising an additional long amino acid amino sequence (eg 11 or more amino acids) at the Nor C-terminal may be considered as a MIC-1 conjugate. MIC-1 conjugates suitable for use as described herein may comprise, for example, a conjugate of a MIC-1 with a conjugate partner selected from the group comprising fusion partners to improve protein recovery or expression (eg Human serum albumin (HSA), Glutathione S-transferase (GST), the pro-region of another member of the TGF-β superfamily, and various affinity-tags such as a polyhistidine tag (His-tag) or FLAG-tag), and Fc polypeptides and other polypeptides capable of dimerising. The MIC-1 molecule of a MIC-1 conjugate may optionally be conjugated to the conjugate partner via a linker sequence as is well known to those skilled in the art.

Some particular examples of suitable MIC-1 conjugates are described in International patent publication No WO 2015/197446. These conjugates comprise the fusion partner HSA (or a functional variant thereof) at the N-terminus conjugated via a linker sequence to a MIC-1 comprising a native MIC-1 or variant MIC-1 amino acid sequence at the C-terminus of the conjugate. The peptide linker is 10 to 50 amino acids in length and comprises the amino acid sequence [X-Yₘ]ₙ, wherein X is Asp or Glu, Y is Ala, m is from 2 to 4, and n is at least 2.

In some embodiments, the first agent comprises a MIC-1 conjugate that is a conjugate of MIC-1 and one or more Fc polypeptides. The term "Fc polypeptide" will be well understood by those skilled in the art as referring to the non-antigen binding portion or "crystallisable fragment" of an immunoglobulin or antibody, or fragment thereof, through which an antibody is able to mediate effector functions, such as binding to a cellular receptor and inducing immune responses. The Fc conjugate partner may enable the formation of a dimer of the MIC-1 conjugate by dimerisation between Fc polypeptides. Some particular examples of MIC-1-Fc polypeptide conjugates are described in International patent publication No WO 2013/113008.

In embodiments where a first agent comprises recombinant MIC-1, preferably the recombinant MIC-1 is recombinant human MIC-1 (rhMIC-1), preferably in dimeric form, or a conjugate of rhMIC-1 (eg a conjugate of rhMIC-1 with an Fc polypeptide in dimeric form).

Described herein is a MIC-1 agonist. As used herein, the term "MIC-1 agonist" is to be understood as referring to any agent that binds to and activates a MIC-1 receptor^{30,69,70} to produce, at least partially, a biological response or activity of MIC-1 (eg agents which
mimic an activity of MIC-1). Suitable MIC-1 agonists may, for example, stimulate glycosaminoglycan and collagen production in simple assays such as those described in WO 97/000958 *supra* (where an observed increase in the production of these matrix proteins indicates MIC-1 agonist activity). Additionally or alternatively, suitable MIC-1 agonists may be identified by, for example, bioassay (eg by subcutaneously administering the agent to mice and monitoring body weight and food intake to confirm that they are decreased). Examples of such bioassays are described in WO 2015/197446 *supra.* Another approach to identifying suitable MIC-1 agonists involves the incubation of the agent with a cell line stably expressing the MIC-1 receptor and co-receptor RET and detecting an increase in phosphorylation levels of any one or more of RET, extracellular signal-regulated kinase (ERK) and the serine/threonine-specific protein kinase AKT⁶⁹. Further bioassays that are useful in identifying and/or assessing suitable MIC-1 agonists are described in International patent publication No WO 2017/121865.

As such, the term "MIC-1 agonist" encompasses agents comprising an active MIC-1 fragment, analogues and peptide mimetics of the active domains of MIC-1, and small organic molecules which mimic MIC-1 activity. Active MIC-1 fragments may be conjugated with a conjugate partner selected from the group comprising fusion partners to improve protein recovery or expression, and Fc polypeptides and other polypeptides capable of dimerising.

In some embodiments, the first agent comprises an active MIC-1 fragment. Suitable active MIC-1 fragments preferably comprise a fragment of MIC-1 that retains the characteristic and highly conserved seven-cysteine domain of members of the TGF-β superfamily (which spans about 80 amino acids and encompasses most of the mature form of the respective proteins) but which lacks one or more of the N-terminal sequence amino acids of the mature MIC-1 protein (eg amino acid (aa) 1-13); that is, an N-terminal truncation. Particular examples include (Δ1-13)MIC-1 (where all of the 13 N-terminal amino acids have been deleted), (Δ1-10)MIC-1, (Δ1-5)MIC-1, (Δ1-3)MIC-1 (specifically described in the abovementioned WO 2017/202936) and (Δ1)MIC-1 (where only the N-terminal amino acid of the mature MIC-1 protein (alanine (Ala) in mature human MIC-1) is deleted). An active MIC-1 fragment may comprise an active variant MIC-1 amino acid sequence as described above, but preferably, comprises an active fragment of a MIC-1 comprising a native MIC-1 amino acid sequence. Active MIC-1 fragments suitable for use in the method of the first aspect can be readily produced by those skilled in the art by using, for example, standard recombinant or protein synthesis techniques. A first agent comprising an active MIC-1 fragment may further comprise a conjugate partner selected from the group comprising fusion partners to improve protein recovery or expression, and Fc polypeptides and other polypeptides capable of dimerising.

Described herein is a MIC-1-inducing agent. As used herein, the term "MIC-1-inducing agent" is to be understood as referring to any agent that induces, directly or indirectly, expression of endogenous MIC-1 in a subject. Suitable MIC-1-inducing agents may, for example, be identified by simple MIC-1 expression assays such as those described in WO 97/000958 *supra* involving the culture of macrophages (eg peripheral blood derived macrophages), monocytoid cell lines (eg U937) and/or human neonatal fibroblast cell lines (eg CCD34Lu) with the agent and determining increased expression of MIC-1 mRNA and/or MIC-1 protein.

As such, the term "MIC-1-inducing agent" encompasses agents which enhance transcription or translation of the *MIC-1* gene such as the p53 transcription factor (which is often seen in elevated levels in diseases associated with MIC-1 over-expression), factors which enhance p53 expression or activity (eg nutlin⁶²), non-steroidal anti-inflammatory drugs (NSAIDS) known to induce MIC-1 expression (eg sulindac sulfide and other NSAIDS that induce expression of the EGR-1 transcription factor⁶³⁻⁶⁵), and various anti-tumourigenic compounds (eg resveratrol, genistein, diallyl disulfide, retinoid 6-[3-(1-adamantyl)-4-hydroxyphenyl]-2-naphthalene carboxylic acid, 2-(4-amino-3-methylphenyl)-5-fluorobenzothiazole, and peroxisome proliferator-activated receptor-γ ligands⁶³).

As will be well known to those skilled in the art, a first agent comprising MIC-1 and/or an active MIC-1 fragment as described above (particularly, a first agent that comprises an active MIC-1 fragment) may also comprise other compounds, complexing agents and substances which may be covalently or non-covalently linked, which may confer various additional functionalities or properties, such as improved bioavailability. For example, a first agent may comprise MIC-1 and/or an active MIC-1 fragment that is covalently or non-covalently linked to polyethylene glycol polymer chains (eg PEGylation) and various well known absorption enhancers (particularly for improving oral bioavailability) including, for example, fatty acids and their derivatives (eg oleic acid, linoleic acid, caprylic acid, capric acid, mono and diglycerides, and acylcarnitines such as lauroyl-L-carnitine chloride and palmitoylcarnitine chloride), cationic polymers (eg chitosan and its derivatives) and anionic polymers (eg carbopol and polyacrylic acid derivatives, and N-acetyl cysteine), and monomers for the formation of hydrogels (eg 2-hydroxyethyl methacrylate, ethylene glycol dimethacrylate, N-isopropyl acrylamide, acrylic acid and methacrylic acid).

Notwithstanding the above, a first agent comprising MIC-1 and/or an active MIC-1 fragment suitable for use according to the invention may additionally or alternatively, comprise amino acid sequences that have been modified either by natural processes, such as post-translational processing, or by chemical modification techniques such as those well known to those skilled in the art. Such modifications can occur anywhere in the molecule, including for example, within the peptide backbone, the amino acid side-
chains and/or the C-terminal. It will also be appreciated that the same types of modifications may be present in the same or at varying degrees at several sites in the molecule. One particular example of chemical modification is C-terminal amidation. Accordingly, the MIC-1 and/or an active MIC-1 fragment of the first agent may have an amide group at the C-terminal. Methods for the amidation of the C-terminal of a polypeptide (eg α-amidation) are well known to those skilled in the art. The C-terminal amide may avoid carboxypeptidase degradation of the MIC-1 and/or an active MIC-1 fragment.

The composition according to the invention may involve a second agent that comprises a recombinant leptin or synthetic leptin (ie leptin produced by protein synthesis techniques) comprising a native leptin amino acid sequence (eg the native amino acid sequence of the 146 aa mature human leptin protein) or a variant leptin amino acid sequence. Suitable variant leptin amino acid sequences may include a naturally occurring or non-natural variant amino acid sequence of a native amino acid sequence that may include one or more minor sequence variations which, preferably, do not substantially alter the function of the polypeptide (eg despite the variation(s), the polypeptide maintains the ability of binding to and activating a leptin receptor (LEP-R, but also known as OB-R)). One example of a non-natural variant of leptin is the leptin W100E variant (comprising a Trp→Glu substitution at position 100)⁵¹. Other variants of leptin amino acid sequences may include one or more conservative amino acid substitutions such as: G, A, V, I, L, M; D, E; N, Q; S, T; K, R, H; F, Y, W, H; and P, Nα-alkylamino acids. Other substitutions may include the substitution of one or more L-amino acid(s) with a D-amino acid(s). Preferably, any amino acid substitution comprises a substitution with an amino acid selected from the twenty (20) standard amino acids encoded by the genetic code (ie the canonical amino acids). However, amino acid substitutions with non-canonical amino acids such as those mentioned above at paragraph [0047], are also contemplated. Other sequence variations that may be present include one or more amino acid deletion or addition (eg insertion). Other additions that may be made to, for example, the N- or C-terminal sequence may comprise the addition of a single amino acid (eg Ala or Met; nb. an N-methionyl residue is included in one well known analogue of human leptin, namely N-methionyl leptin (metrepletin) which is considered to be suitable for use as described herein), short amino acid sequences (eg 2 to 10 amino acids in length) or long amino acid sequences (eg 11 or more amino acids) which may confer various additional functionalities or properties, such as improved bioavailability, protein recovery or expression (eg a fusion partner). Variant leptin agents comprising an additional long amino acid amino sequence (eg 11 or more amino acids) at the N- or C-terminal may be considered as a leptin conjugate. Leptin conjugates suitable for use as described herein may comprise, for example, a conjugate of a leptin with a conjugate partner selected from the group comprising fusion partners to improve protein recovery or expression (eg Human serum albumin (HSA), Glutathione S-transferase (GST), and various affinity-tags such as a polyhistidine tag (His-tag) or FLAG-tag). The leptin molecule
of a leptin conjugate may optionally be conjugated to the conjugate partner via a linker sequence as is well known to those skilled in the art.

In embodiments where a second agent comprises recombinant leptin, preferably the recombinant leptin is recombinant human leptin (rhLeptin) or a conjugate of rhLeptin (eg a conjugate of rhLeptin with HSA).

Described herein is a leptin agonist. As used herein, the term "leptin agonist" is to be understood as referring to any agent that binds to and activates a leptin receptor to produce, at least partially, a biological response or activity of leptin (eg agents which mimic an activity of leptin). Suitable leptin agonists may be readily assayed to determine their ability to bind to and activate a leptin receptor (LEP-R) using a cellular assay as described in, for example, International patent publication No WO 2009/108340, wherein a cell line expressing LEP-R will be stimulated to grow as a result of treatment with an agent with leptin agonist activity. Alternatively or additionally, suitable leptin agonists may be identified by bioassay involving determining their ability to substitute for leptin in leptin-deficient animals.

As such, the term "leptin agonist" encompasses agents comprising an active leptin fragment, analogues and peptide mimetics of the active domains of leptin such as the 12-residue glycosylated leptin-based peptidomimetic E1/6-amino-hexanoic acid (Aca)⁵² and other molecules based upon the third receptor-binding region ("site III") of leptin, and small organic molecules which mimic leptin activity. Active leptin fragments may be conjugated with a conjugate partner selected from the group comprising fusion partners to improve protein recovery or expression.

In some embodiments, the second agent comprises an active leptin fragment. Suitable leptin fragments may include leptin 22-56, leptin 57-92³⁵, leptin 93-122⁴³ and leptin 116-130³⁶. Further examples of active leptin fragments which may be suitable for use as described herein have been described in International patent publication No WO 96/34885 and include fragments of leptin comprising amino acids 26 to 39 (ie leptin 26-39), 74 to 88, 93 to 113 or 142 to 161. An active leptin fragment may comprise an active variant leptin amino acid sequence as described above, but preferably, comprises an active fragment of a leptin comprising a native leptin amino acid sequence. Active leptin fragments suitable for use as described herein can be readily produced by those skilled in the art by using, for example, standard recombinant or protein synthesis techniques. A second agent comprising an active leptin fragment may further comprise a conjugate partner selected from the group comprising fusion partners to improve protein recovery or expression.

As will be well known to those skilled in the art, a second agent comprising leptin and/or an active leptin fragment as described above (particularly, a second agent that comprises an active leptin fragment) may also comprise other compounds, complexing agents and substances which may be covalently or non-covalently linked, which may confer various additional functionalities or properties, such as improved bioavailability. For example, a second agent may comprise leptin and/or an active leptin fragment that is covalently or non-covalently linked to polyethylene glycol polymer chains (eg PEGylation) and various well known absorption enhancers (particularly for improving oral bioavailability) including, for example, fatty acids and their derivatives, cationic polymers and anionic polymers, and monomers for the formation of hydrogels. In addition, and notwithstanding the above, a second agent comprising leptin and/or an active leptin fragment suitable for use as described herein may additionally or alternatively, comprise amino acid sequences that have been modified either by natural processes, such as post-translational processing, or by chemical modification techniques such as those well known to those skilled in the art. Such modifications can occur anywhere in the molecule, including for example, within the peptide backbone, the amino acid side-chains and/or the C-terminal. It will also be appreciated that the same types of modifications may be present in the same or at varying degrees at several sites in the molecule. One particular example of chemical modification is C-terminal amidation (ie to provide the leptin and/or an active leptin fragment with a C-terminal amide group) to prevent carboxypeptidase degradation.

In one particular embodiment, the composition comprises a first agent that comprises MIC-1 or a MIC-1 conjugate, and a second agent that comprises leptin or a leptin conjugate.

As mentioned above, the composition according to the invention may achieve body weight control in an overweight or obese subject. However, the composition may also be useful for a subject who might otherwise desire weight loss for reasons of well-being or vanity.

The composition according to the invention may be particularly useful for an overweight or obese subject who is suffering Type 2 diabetes (T2D). As is well known, T2D is a major complication of obesity and several epidemiological studies have linked MIC-1 to diabetes and insulin resistance. For example, increased serum MIC-1 levels have been found to independently predict the presence of insulin resistance^{28,39} as well as progress to Type 2 diabetes⁴⁰⁻⁴². Thus, it is expected that the administration of a first agent comprising MIC-1 and/or an active MIC-1 fragment along with a second agent comprising leptin and/or an active leptin fragment, will provide a useful therapy for diabetes and/or one or more complications of T2D, obesity or overweight (eg non-alcoholic steatohepatitis (NASH) or glucose intolerance) both because of body weight reduction and the direct effects of MIC-1 (or the MIC-1 agonist) on glucose tolerance and homeostasis.

Typically, the subject will be a human. However, the composition according to the invention may also be applicable to non-human subjects such as, for example, livestock (eg cattle, sheep and horses), exotic animals (eg tigers, lions, elephants and the like) and companion animals (such as dogs and cats).

The composition according to the invention also relates to the use of:
(i) a first agent comprising MIC-1 and/or an active MIC-1 fragment; and
(ii) a second agent comprising leptin and/or an active leptin fragment;
for use in controlling body weight (eg achieving weight loss) and/or appetite in a subject.

Described herein is a pharmaceutical composition comprising:
(i) a first agent comprising MIC-1 and/or an active MIC-1 fragment; and
(ii) a second agent comprising leptin and/or an active leptin fragment.

The pharmaceutical composition may be suitable for, for example, oral, buccal, nasal, intramuscular and intravenous administration. The composition may further comprise, for example, a pharmacologically acceptable carrier and/or excipient, optionally together with others substances such as absorption enhancers and protease inhibitors for improving bioavailability, especially oral bioavailability. The composition may be intended for single daily administration, multiple daily administration, or controlled or sustained release, as needed to achieve the most effective results.

The determination by the present Applicant that treatment with MIC-1 and leptin caused significantly greater weight loss in an obese mouse model than was observed with MIC-1 or leptin alone (see Examples 2 and 3 hereinafter), suggests that where a subject (eg an overweight or obese subject) has a higher than normal serum leptin level (ie a serum leptin level that is typical of most obese subjects and is therefore higher than the typical range for a normal non-obese subject), then it may be sufficient, in a method of controlling body weight and/or appetite in a subject, to administer only the first agent comprising MIC-1, a MIC-1 agonist and/or MIC-1-inducing agent (that is, there may be no need to administer the second agent comprising leptin and/or a leptin agonist).

The composition according to the invention may be for use in a method of controlling body weight (eg achieving weight loss) and/or appetite in a subject according to the invention, said method comprising the steps of:
determining whether said subject has a low, normal or high serum level of leptin; and
administering to said subject, where determined as having a low or normal serum level of leptin, an effective amount of the composition according to the invention comprising:
   (i) a first agent comprising MIC-1 and/or an active MIC-1 fragment; and
   (ii) a second agent comprising leptin and/or an active leptin fragment.

As mentioned above, for normal (ie non-obese) human subjects, the normal serum level range of leptin is broad; for example, about 3.5 to 14.0 ng/ml for adult women and 4.5 to 25.5 ng/ml for adult men after overnight fasting. Thus, a relatively high serum leptin level for a normal human subject may be regarded as a level at the higher end of these ranges (eg more than about 10 ng/ml for adult women and more than about 18 ng/ml for adult men). Such higher than normal serum leptin levels may be caused by, for example, impairment of leptin regulation associated with certain diseases and conditions such as chronic inflammatory diseases (such as inflammatory bowel disease, inflammatory nephritis, pelvic endometriosis, non-alcoholic hepatitis, chronic pulmonary inflammation, Behcet's disease and Graves' disease³⁷) and some cancers (such as breast cancer³⁸). On the other hand, for an obese human subject, the serum leptin level would normally be expected to be high (ie as described above at paragraph [0041]), and a relatively low serum leptin level for an obese human subject (after overnight fasting) may therefore be, for example, for an adult woman, less than about 30 ng/ml or, more preferably, less than 25 or less than 20 ng/ml, and for an adult man, less than about 20 ng/ml or, more preferably, less than 15 ng/ml.

The step of determining the relative level of serum leptin may comprise measuring serum leptin levels in accordance with any suitable assay method known to those skilled in the art including standard immunoassays (eg a leptin ELISA).

The composition according to the invention comprising:
(i) a first agent comprising MIC-1 and/or an active MIC-1 fragment; and
(ii) a second agent comprising leptin and/or an active leptin fragment;
may be for use in controlling body weight (eg achieving weight loss) and/or appetite in a subject according to the invention determined as having a low or normal serum level of leptin.

The present invention provides a kit comprising first and second containers
(eg vials), wherein the first container contains a first agent comprising MIC-1 and/or an active MIC-1 fragment, and the second container contains a second agent comprising leptin and/or an active leptin fragment; optionally packaged with instructions for the use according to the invention.

The first and second agents provided in the containers of the kit may be as described above. The kit may further comprise materials and agents for assaying MIC-1 and/or leptin as described above.

The present Applicant has previously described methods for treating anorexia or cachexia by administering to a subject suffering from such condition a MIC-1-inhibiting agent such as an antagonistic anti-MIC-1 monoclonal antibody^{21, 22}. It is considered that the finding described hereinafter in the Examples, that treatment with MIC-1 and leptin caused significantly greater weight loss in an obese mouse model than was observed with MIC-1 or leptin alone, is also of relevance to the treatment of anorexia and cachexia. Further, it was found that when administered to normal (non obese) chow fed mice, the combination of MIC-1 and leptin caused a much greater loss of lean mass which is considered especially important in the pathogenesis of anorexia/cachexia syndromes. Thus, it is expected that treatment of an anorexic/cachectic subject with agents to inhibit both MIC-1 and leptin, will achieve significantly improved results (ie in the form of weight gain or increase in fat and lean mass, or at least, a reduction in any further loss of body weight or lean or fat mass) than that achieved by treatment with a MIC-1-inhibiting agent alone.

Described herein, but not part of the invention, is a method of increasing body weight and/or appetite in a subject, said method comprising administering to said subject an effective amount of:
(i) a MIC-1-inhibiting agent; and
(ii) a leptin-inhibiting agent.

The method described herein, but not part of the invention, may be conducted to achieve an increase in body weight and/or appetite or, at least, control of body weight and/or appetite in a subject. For example, the method may be conducted to achieve body weight gain in a subject or, at least, a reduction in any loss of body weight (eg to prevent further weight loss in an anorexic/cachectic subject). The subject may be, for example, a subject suffering from anorexia or cachexia. Cachectic subjects may be subjects suffering from decreased appetite and/or weight loss associated with inflammatory disease (eg rheumatoid arthritis), chronic renal and/or cardiac failure, and/or cancer (particularly, an epithelial cancer such as breast, prostate, colonic, rectal, bladder, lung and pancreatic cancer). The method may, however, also be useful for the treatment of decreased appetite and/or weight loss associated with any other disease, condition or treatment wherein MIC-1 is over-expressed (eg injury, inflammation, stress, and radiotherapy and chemotherapy). Subjects suitable for treatment may therefore show MIC-1 over-expression or, at least, a serum level of MIC-1 that is consistently at the high end of the normal serum level of 0.15-1.15 ng/ml. Such subjects can be determined by the detection of a relatively high serum MIC-1 level (eg from a whole blood or serum sample) using any suitable assay for MIC-1 known to those skilled in the art including standard immunoassays (eg a MIC-1 ELISA⁴). Additionally, subjects suitable for treatment may show a relatively high serum level of leptin. For example, a serum level of leptin at the higher end of the normal ranges mentioned above (eg more than about 10 ng/ml for adult women and less than about 18 ng/ml for adult men). As mentioned above, the relative level of serum leptin may be determined by measuring serum leptin levels in accordance with any suitable assay method known to those skilled in the art including standard immunoassays (eg a leptin ELISA).

The subject of the method described herein, but not part of the invention, may be a cachectic subject suffering from decreased body weight and/or appetite associated with advanced cancer, where a high total tumour mass often leads to a high serum level of MIC-1. Leptin is also commonly over-expressed in cancerous tissues, especially those of the pancreas, colon, lung, breast, ovary and prostate (nb. the searchable database, *The Human Protein* Atlas, reveals that leptin is over-expressed at both the mRNA
and protein levels in a significant proportion of patients with malignant tumours in these organs). Also, as mentioned above, some cancers (eg breast cancer) are known to cause higher than normal serum leptin levels; however, since leptin is prominently or mainly expressed in adipose tissue (ie such that leptin levels may, at least in part, reflect the fat mass of a subject) which is typically rapidly lost in cachexia, the effect of a leptin-expressing tumour on the serum leptin level may be greatly offset by the reduced level of leptin expression from adipose tissue (ie due to loss of adipose tissue). Thus, a cachectic subject with advanced cancer may or may not also show a high serum level of leptin, but typically, will at least show a higher serum leptin level than that of other age-, height- and gender-matched subjects with a comparable degree of adiposity.

The MIC-1-inhibiting agent described herein, but not part of the invention, may decrease the amount of MIC-1 (ie endogenous MIC-1) in the subject (particularly, the serum level of MIC-1) and/or decrease the activity of MIC-1 in the subject.

The leptin-inhibiting agent described herein, but not part of the invention, may decrease the amount of leptin (ie endogenous leptin) in the subject (particularly, the serum level of leptin) and/or decrease the activity of leptin in the subject.

The MIC-1-inhibiting agent may, for example, be administered to the subject concurrently (eg in combination) with the leptin-inhibiting agent, or otherwise the MIC-1-inhibiting agent and the leptin-inhibiting agent may be administered to the subject consecutively and in either order. As such, when administered consecutively, the respective agents may be administered one after another with practically no time interval (ie one is administered effectively immediately after the other) or, otherwise, after an interval of, for example, 1 to 5 minutes, 10 minutes, 30 minutes, 60 minutes, 4 hours or 12 hours or more. Where the MIC-1-inhibiting agent and leptin-inhibiting agent are administered consecutively, they may each be formulated for administration in a pharmaceutical composition form including a pharmacologically acceptable carrier and/or excipient, which may be the same or different. The pharmaceutical compositions may be suitable for, for example, oral, buccal, nasal, intramuscular and intravenous administration. The pharmaceutical compositions may optionally comprise other substances such as absorption enhancers (including surfactants) and chelating agents. Typically, the pharmaceutical compositions will be administered to the subject in an amount which is effective for increasing body weight and/or appetite. The composition comprising the MIC-1-inhibiting agent may be administered to provide, for example, an amount of the agent that is between about 0.01 and about 100 mg/kg body weight per day, or between about 0.05 and 25 mg/kg body weight per day of the agent. The composition comprising the leptin-inhibiting agent may be administered to provide, for example, an amount of the agent that is between about 0.01 and about 1000 mg/kg body weight per day, or between about 1 and 750 mg/kg body weight per day of the agent. However, it will be understood by those skilled in the art that the administered amount of the pharmaceutical compositions, and the frequency of administration for any particular subject, may vary and depend upon a variety of factors including the activity of the active agents (eg the MIC-1-inhibiting agent and the leptin-inhibiting agent), the metabolic stability and length of action of the active agents, the age, body weight, sex, mode and time of administration, and the rate of excretion of the active agents. The pharmaceutical compositions may be intended for single daily administration, multiple daily administration, or controlled or sustained release, as needed to achieve the most effective results.

The MIC-1-inhibiting agent may comprise an antagonistic anti-MIC-1 antibody (ie a neutralising MIC-1 antibody) or a fragment thereof (eg MIC-1-binding fragments including those selected from Fab fragments, recombinant scFv fragments⁴⁴ and single-domain (sdAb) antibodies), catalytic or inhibitory oligonucleotide molecule targeted against the *MIC-1* gene (eg a ribozyme, DNAzyme, antisense RNA, or small inhibitory RNA (siRNA)), inhibitor of *MIC-1* transcription or translation, a soluble extra-cytoplasmic receptor domain of the MIC-1 receptor^{30, 69, 70}, or a peptide or small organic molecule which inhibits, for example, MIC-1 binding to its receptor, MIC-1 receptor phosphorylation, or MIC-1 receptor signalling. One example of a soluble extra-cytoplasmic receptor domain of the MIC-1 receptor that may be suitable for use as a MIC-1-inhibiting agent is a protein expressed by an alternative murine mRNA splice variant denoted GRAL-B⁷², or a human ortholog thereof; which lack a transmembrane and cytoplasmic domain. Suitable MIC-1-inhibiting agents may be assayed by one or more simple assays relevant to the intended mechanism of inhibition. For example, antagonistic anti-MIC-1 antibodies may be assessed for their ability to bind to MIC-1 using standard immunoassays and/or inhibit (antagonise) MIC-1 activities observed in the assays and bioassays described above at paragraph [0051]. For some other MIC-1-inhibiting agents it may be more relevant to determine inhibition of expression of MIC-1 mRNA or MIC-1 protein in the cellular assays described in paragraph [0054] above.

The MIC-1-inhibiting agent described herein, but not part of the invention, may comprise an antagonistic anti-MIC-1 antibody or a fragment thereof. Preferably, the MIC-1-inhibiting agent comprises a human anti-MIC-1 antibody (such as may be produced by well known techniques using transgenic mice or phage display^{45,46}) or a chimeric or humanised anti-MIC-1 antibody (such as may be produced by well known techniques described in, for example, US Patent No. 5,225,539, by specificity determining residue (SDR) grafting⁴⁷, by affinity maturation using phage display⁴⁸, or using heavy chain complementarity-determining region 3 grafting coupled with *in vitro* somatic hypermutation⁴⁹).

The leptin-inhibiting agent described herein, but not part of the invention, may comprise an antagonistic anti-leptin antibody (ie a neutralising leptin antibody) or a fragment thereof (eg leptin-binding fragments including those selected from Fab fragments, recombinant scFv fragments and single-domain (sdAb) antibodies), a catalytic or inhibitory oligonucleotide molecule targeted against the leptin (*ob* or *lep*) gene (eg a ribozyme, DNAzyme, antisense RNA, or small inhibitory RNA (siRNA)), an inhibitor of *ob* transcription or translation (eg
silibinin and curcumin⁵⁰), a soluble extra-cytoplasmic receptor domain of the leptin receptor (eg the LEP-R:Fc fusion protein described by De Rosa *et al.*⁵⁵), an antagonistic anti-leptin receptor antibody (ie a neutralising anti-LEP-R antibody⁵⁴ ) or a fragment thereof (eg leptin receptor-binding fragments including those selected from Fab fragments, recombinant scFv fragments and single-domain (sdAb) antibodies), or a leptin mutant, peptide or small organic molecule which inhibits, for example, leptin binding to its receptor, leptin receptor phosphorylation, or leptin receptor signalling (eg by binding, but not activating, the leptin receptor). One suitable example of a leptin mutant includes an Arg→Glu substitution at position 128 that abolishes biological activity without affecting receptor binding⁵³. Another suitable example of a leptin mutant is a dominant negative form of leptin^{66, 74}. Suitable leptin-inhibiting agents may be assayed by one or more simple assays relevant to the intended mechanism of inhibition. For example, antagonistic anti-leptin antibodies may be assessed for their ability to bind to leptin using standard immunoassays and/or inhibit (antagonise) leptin activities observed in the assays and bioassays described above at paragraph [0060]. For some other leptin-inhibiting agents it may be more relevant to determine inhibition of expression of leptin mRNA or leptin protein in suitable cellular assays.

The leptin-inhibiting agent described herein, but not part of the invention, may comprise an antagonistic anti-leptin antibody or a fragment thereof. Preferably, the leptin-inhibiting agent comprises a human anti-leptin antibody, or a chimeric or humanised anti-leptin antibody. Such antibodies may be produced using, for example, any of the well known methods described in paragraph [0088] above.

Described herein, but not part of the invention, is the use of:
(i) a MIC-1-inhibiting agent; and
(ii) a leptin-inhibiting agent;
for increasing body weight and/or appetite in a subject.

Described herein, but not part of the invention, is a pharmaceutical composition comprising:
(i) a MIC-1-inhibiting agent; and
(ii) a leptin-inhibiting agent.

The pharmaceutical composition may be suitable for, for example, oral, buccal, nasal, intramuscular and intravenous administration. The composition may further comprise, for example, a pharmacologically acceptable carrier and/or excipient, optionally together with others substances such as absorption enhancers and protease inhibitors for improving bioavailability, especially
oral bioavailability. The composition may be intended for single daily administration, multiple daily administration, or controlled or sustained release, as needed to achieve the most effective results.

Described herein, but not part of the invention, is a kit comprising first and second containers (eg vials), wherein the first container contains a MIC-1-inhibiting agent and the second container contains a leptin-inhibiting agent; optionally packaged with instructions for the use of the kit as described herein.

The MIC-1-inhibiting agent and leptin-inhibiting agent provided in the containers of the kit may be as described above. The kit may further comprise materials and agents for assaying MIC-1 and/or leptin as described above.

The disclosure is hereinafter expanded by way of the following non-limiting examples and accompanying figures.

### EXAMPLES

### Example 1 The effect of MIC-1 on weight loss in obese mice

A study was conducted using groups of ten (10) male 22 week old C57BL6 mice, CHOW fed or with diet-induced obesity (DIO) from 12 weeks of a high fat diet (HFD). The mice were infused via osmotic minipump (ag Alzet^{®} model 2002; Durect Corporation, Cupertino, CA, United States of America) with either vehicle (CHOWc or DIOc) or recombinant murine MIC-1 (0.5 µg/gBW/day; CHOWm or DIOm) produced in accordance with a previously described method²¹. It was found that MIC-1 induced sustained weight loss, accompanied by a decrease in food intake (Figures 1A and B) which was proportionately about twice as great in the HFD (14%) mice than in CHOW fed mice (7%; Figure 1A), despite having much lower steady state serum MIC-1 levels (respectively 6.3+/-0.8ng/ml vs 10.7+/-1.3ng/ml; p< 0.01). As measured by standard dual-energy X-ray adsorptiometry (DEXA), over the course of the study, DIOm mice lost a substantial amount of fat mass but little lean mass compared to the CHOWm mice that lost both fat and lean mass (Figure 1C and D). Further, the inguinal fat tissue of DIOm mice showed much less normalised qPCR expression of the macrophage marker F4/80 (10.3+/-1.9 vs 73.2+/-10.5; p<0.05), indicating a decrease in macrophage fat infiltration, which would be expected to improve the metabolic phenotype⁷².

The data obtained in this study therefore indicates that MIC-1 has very favourable characteristics for its use as an anti-obesity agent. In particular, the study found that MIC-1 is much more effective in obese than lean mice and, in further support of this, also found that the rate of weight loss in the obese mice becomes progressively less as those mice lost weight and it causes insignificant loss of lean mass. In similar experiments⁷² conducted over a period of 34 days (ie prolonged infusion with recombinant MIC-1 for 34 days) with mice fed on normal chow or HFD for 16 weeks in order to induce DIO, it was also observed that MIC-1 decreased food intake and body weight of DIO mice and chow fed mice compared with vehicle treated control mice. In the DIO mice, these effects were also accompanied by a greater reduction in fat mass. Further, whilst MIC-1 treated chow fed mice lost lean as well as fat-mass, the MIC-1 treated DIO mice lost fat mass alone. The reduction in body weight and adiposity was due largely to reduced food intake, but MIC-1 treated DIO mice also displayed increased energy expenditure that may be due to increased thermogenesis. In addition, the MIC-1 treated DIO mice showed higher circulating levels of adiponectin and lower tissue expression and circulating levels of leptin and inflammatory mediators associated with insulin resistance, and displayed marked improvement in the steatohepatitis of obesity. Peripheral insulin and glucose intolerance were improved in both MIC-1 treated DIO and normal chow fed mice compared to vehicle-treated control mice.

### Example 2 Increased weight loss in mice infused with MIC-1 plus leptin

A study was conducted using chow fed 11 week old C57BL6 mice continuously infused (using a standard osmotic minipump) with either vehicle, recombinant murine MIC-1 (0.5 µg/g body weight/24hrs), leptin (0.5 µg/g body weight/24hrs), or MIC-1 plus leptin (0.5 µg/g body weight MIC-1 + 0.5 µg/g body weight leptin/24hrs) for six (6) days. The leptin was recombinant murine leptin sourced from Creative Biomart (Shirley, NY, United States of America).

The results are shown in Figure 2. After 6 days, the body weight of the mice treated with MIC-1 plus leptin was significantly less than that observed with MIC-1 or leptin alone, indicating a substantial complementary interaction between the two adipokines. Moreover, as the DIO mice used in Example 1 are leptin-resistant, the results achieved here indicate that MIC-1 may also mitigate leptin resistance. This is also consistent with previous work by the present Applicant which showed that MIC-1 was effective in causing anorexia and weight loss in leptin-deficient ob/ob mice and leptin receptor-deficient db/db mice²¹.

The data obtained in this study also indicates that MIC-1 has very favourable characteristics for its use as an anti-obesity agent, particularly when used in combination with leptin. It is considered that prolonged use of MIC-1 and/or leptin will lead to sustained weight loss and correct the metabolic consequences of diet-induced obesity (DIO).

### Example 3 Substantially increased weight loss in HFD mice infused with MIC-1 plus leptin

Vehicle, murine leptin (0.5 µg/g/day) and recombinant murine MIC-1 (0.5 µg/g/day) were infused in binary combinations into normal chow fed male mice and male mice rendered obese (mice with diet-induced obesity (DIO)) and leptin-resistant after 24 weeks on a high fat diet. The results are shown in Figures 3A and 3B. The greatest levels of weight loss were found when mice were infused with the combination of MIC-1 and leptin. For the HFD mice, the degree of weight loss observed with the combination of MIC-1 and leptin was particularly pronounced.

The results confirm that HFD mice are more responsive to MIC-1-induced weight loss than chow fed mice. In particular, it was observed in this study that the HFD mice lost proportionately more weight and continued to lose weight whilst the chow fed mice weight loss plateaued by about 11 days. In addition, the results showed that when using doses of leptin that are equipotent with MIC-1 in inducing weight loss in chow fed mice (see Figure 3B), there is little or no leptin-induced weight loss in HFD mice (Figure 3A). In contrast, when leptin is infused with MIC-1 in the HFD mice, the degree of weight loss is substantially increased, and even at the end of the experiment, the mice treated with the combination of MIC-1 and leptin continued to rapidly lose weight (Figure 3A).

### Example 4 Characterisation of increased weight loss in HFD mice infused with MIC-1 plus leptin

Further experiments were conducted to demonstrate and characterise an interaction between MIC-1 and leptin in mice with diet induced obesity (DIO) mice and normal chow fed mice, using 7 mice per group. The methods used were similar to those described in relation to Examples 1-3 and/or were as previously described⁷¹. Each mouse was implanted with two osmotic minipumps (Alzet model 2002; Durect Corporation). One pump contained either vehicle or MIC-1 (recombinant mature domain of murine MIC-1 expressed and secreted from *P. pastoris* grown in minimal media and highly purified in a 3 step procedure, then lyophilised and stored at -80°C until use, at which time it was reconstituted in 4mM HCL then diluted to the appropriate concentration in PBS vehicle) and the second pump contained either vehicle or leptin (recombinant murine leptin produced in *E.coli* as a single, non-glycosylated, polypeptide chain consisting of 147 aa's, with a molecular weight of 16. IkDa). Vehicle or MIC-1 was infused via the minipump at 0.5 µg/gBW/day for the duration of the experiment, while vehicle or leptin was infused at 0.2 µg/gBW/day for the duration of the experiment. Body weight (BW) was monitored for the first 13 days during which mice were largely undisturbed. Whole body fat-mass and lean-mass were measured during treatment using dual-energy X-ray absorptiometry (DEXA; Lunar PIXImus2 mouse densitometer; GE Healthcare, Waukesha, WI, United States of America). Investigations such as DEXA and food intake measurements, which substantially disrupt mouse behaviours, were undertaken between days 15 and 19, over which period body weight was not monitored. Effects on normal liver and liver from DIO were investigated using tissue from dissected livers fixed, sectioned and stained with haematoxylin and eosin. Standard glucose tolerance tests (GTT) and insulin sensitivity tests (ITT) were also performed (at day 14) on the treated mice.

Results are shown in Figures 4 to 10.

As it had been previously demonstrated, it was found that DIO mice treated in these experiments with MIC-1 lost a substantial amount of body weight. On the other hand, DIO mice treated with leptin alone (ie leptin + vehicle) displayed unaltered body weight, which is consistent with well known leptin resistance of severely obese mice and humans. However, when combined with MIC-1, MIC-1 + leptin treated mice lost further body weight over mice treated only with MIC-1 (ie MIC-1 + vehicle), suggesting a synergistic relationship between MIC-1 and leptin (Figure 4). While not wishing to be limited by theory, it is considered that the increase in body weight loss observed with the combination of MIC-1 and leptin may be due to a reversal of leptin resistance by the MIC-1. Food consumption changes followed the changes in body weight indicating that the changes are effected mainly, but not necessarily exclusively, by altered appetite. Similar, though smaller results were observed with normal chow fed mice (see Figure 5) which also exhibited a smaller response to leptin+vehicle as they were not leptin resistant.

It was observed that the body weight lost by the DIO mice treated with MIC-1 or the combination of MIC-1 + leptin was largely fat mass (see Figure 6); whereas these treatments had no impact on lean and muscle masses as determined by analysis of percent change in lean mass (gastrocnemius muscle mass and tibialis anterior muscle mass) quantified by DEXA (Figure 7). This indicates that a treatment of obesity based upon MIC-1 or MIC-1 + leptin would have no unfavourable impact on lean/muscle mass. On the other hand, in normal chow fed mice, infusion with MIC-1 does result in loss of lean mass, and this loss of lean/muscle mass was increased when given in combination with leptin (Figure 8). However, this may mean that in the context of anorexia/cachexia syndromes, treatment with a MIC-1-inhibiting agent (eg an antagonistic anti-MIC-1 antibody) or a combination therapy of a MIC-1-inhibiting agent and a leptin-inhibiting agent (eg an antagonistic anti-MIC-1 antibody with an antagonistic anti-leptin antibody) may bring about an advantageous improvement in the lean/muscle mass of anorexic/cachectic subjects. The normal chow fed mice treated with MIC-1 also lost fat mass, and treatment with MIC-1 + leptin resulted in a significantly greater loss of fat mass (see Figure 10); suggesting that inhibiting leptin or the leptin pathway with a leptin-inhibiting agent will potentiate the effect of inhibiting MIC-1 on the treatment of anorexic/cachectic subjects by improving fat mass as well as lean mass and body weight.

Using liver sections prepared as described above, histology clearly demonstrated the presence of steatohepatitis in vehicle treated DIO mice (*data not shown*) compared to the normal mouse liver. Hepatic steatosis is a major cause of morbidity in obesity and can lead to non-alcoholic steatohepatitis (NASH) and sometimes cirrhosis and liver failure. It was found that the observed steatohepatitis improved over the treatment period in the mice infused with MIC-1 alone (ie MIC-1 + vehicle), and returned essentially to normal with infusion of the combination of MIC-1 and leptin treatment, indicating that these agents offer considerable promise as a combination therapy to treat or prevent steatohepatitis and thereby reduce the risk of development of complicating cirrhosis and liver failure.

With the IGT and ITT, it was found that the combination treatment with leptin potentiated a previously recognised ability of MIC-1 to improve glucose tolerance and insulin sensitivity of obese mice (see Figure 9). Since Type 2 diabetes (T2D) is a major complication of severe obesity that is responsible for substantial morbidity and increased mortality, the use of MIC-1 in combination with leptin may enable better control and perhaps cure of T2D and/or its symptoms.

### Example 5 Substantially increased weight loss in ob/ob mice infused with MIC-1 plus leptin

Ob/Ob mice have a mutant inactive form of leptin and thus these mice have a functionally inactive leptin pathway, which leads to severe, rapidly progressive obesity and its consequences on a normal diet. The mice do, however, have an intact leptin receptor and signalling pathway and can thus respond to the administration of leptin despite not responding to their endogenous mutant leptin. The mice were used in the experimentation here, in order to further examine the interaction between MIC-1 and leptin, in an obesity model without elevated background levels of normal leptin (cf. as occurs in DIO mice). The methods used in the experiments were as described in relation to Examples 1-3 and/or as previously described⁷¹.

Ob/ob genetically leptin deficient mice have no bioactive circulating leptin and are severely obese even on a normal chow diet. They experience some weight reduction following treatment with MIC-1 alone although this is less than that seen with mice that are obese because of a high fat diet (see Figure 11). In the experiments, it was observed that ob/ob mice treated with leptin alone displayed significant weight loss; this is believed to be due to a level of hypersensitivity to leptin because of the genetic deficiency. However, when combined with MIC-1, it was found that mice treated with leptin lose a significant additional amount of body weight over mice treated with either agent alone. Food consumption changes followed the changes in body weight confirming that the changes are effected mainly by altered appetite. It was also found that the ob/ob mice, when treated with MIC-1 alone (ie MIC-1 + vehicle) experience some fat mass reduction (although this is less than that seen with mice that are obese because of a high fat diet (see Figure 12)), but when treated with leptin alone, these mice display significant fat mass loss. Nevertheless, this fat mass reduction is still significantly less than when the ob/ob mice are treated with the combination of MIC-1 and leptin; suggesting a synergistic interaction between the two agents.

### Example 6 The effect of administered leptin on the anorexia/cachexia-inducing activity of MIC-1

The results observed in the previous examples, particularly Example 4, suggested that the anorexia/cachexia-inducing activity of over-expressed MIC-1 could be reduced by inhibiting the leptin pathway. In this study, mice were administered with a leptin antagonist (mouse super leptin antagonist (SMLA); Protein Laboratories Rehevot Ltd, Rehevot, Israel) by continuous infusion. SMLA is a mutant mouse leptin molecule with high affinity for the leptin receptor but which that does not activate the leptin receptor and thus acts as a dominant negative competitive inhibitor of leptin action⁷⁴. The mice were subcutaneously implanted with two osmotic minipumps; one minipump infusing MIC-1 or vehicle (0.5 µg/gBW/day) and the other infusing SMLA or vehicle (1.5 µg/gBW/day). The methods used were as described in relation to Examples 1-3 and/or as previously described⁷¹.

Results are shown in Figures 13-15. Lean mice treated with the leptin antagonist alone displayed increased body weight, due to a reversal of the satiety-inducing effects of leptin in mice and humans. Lean mice treated with MIC-1, mimicking the situation in anorexia/cachexia syndromes, were observed to lose a substantial amount of body weight as previously demonstrated. However, in mice treated with the combination of MIC-1 and the leptin antagonist, less body weight reduction was seen as compared to mice treated with MIC-1 only, indicating that the leptin antagonist inhibits part of MIC-1's action in body weight reduction. It was also observed that these changes in body weight reflect changes in lean mass (Figure 14) and fat mass (Figure 15), thereby indicating that the inhibition of leptin or the leptin pathway reduces the anorexia/cachexia-inducing activity of over-expressed MIC-1. Accordingly, it is anticipated that inhibiting leptin (eg with a leptin-inhibiting agent) in addition to inhibiting MIC-1 may provide an effective therapeutic treatment for a subject suffering from anorexia or cachexia.

Throughout the specification and the claims that follow, unless the context requires otherwise, the words "comprise" and "include" and variations such as "comprising" and "including" will be understood to imply the inclusion of a stated integer or group of integers, but not the exclusion of any other integer or group of integers.

The reference to any prior art in this specification is not, and should not be taken as, an acknowledgement of any form of suggestion that such prior art forms part of the common general knowledge.

It will be appreciated by those skilled in the art that the subject matter described herein is not restricted in its use to the particular application described. Neither is the subject matter restricted in its preferred embodiment with regard to the particular elements and/or features described or depicted herein, but is capable of numerous rearrangements, modifications and substitutions without departing from the scope as set forth and defined by the following claims.

### REFERENCES

1. Bootcov MR et al., Proc Natl Acad Sci USA 94:11514-11519 (1997).
2. Breit SN and MR Bootcov, International Patent Application No PCT/AU96/00386 (WO 97/00958).
3. Fairlie WD et al., Gene 254:67-76 (2000).
4. Moore AG et al., J Clin Endocrinol Metab 85:4781-4788 (2000).
5. Fairlie WD et al., Biochem 40:65-73 (2001).
6. Breit SN et al., International Patent Application No PCT/AU01/00456 (WO 01/81928).
7. Fairlie WD et al., J Leukocyte Biol 65:2-5 (1999).
8. Brown DA et al., Lancet 359:2159-2163 (2002).
9. Koniaris LG. J Gastrointest Surg 2003 7(7):901-905 (2003).
10. Welsh JB and GM Hampton, Cancer Res 61:5974-5978 (2001).
11. Buckhaults P et al., Cancer Res 61:6996-7001 (2001).
12. Welsh JB et al., Proc Natl Acad Sci USA 100:3410-3415 (2003).
13. Brown DA et al., Biotechniques 33(1):118-20, 122, 124 passim (2002).
14. Koopmann J et al., Clin Cancer Res 10(7):2386-2392 (2004).
15. Brown DA et al., Clin Cancer Res 9:2642-2650 (2003).
16. Tsai VWW et al., Int J Obes (Lond) 40(2):193-7 (2016).
17. Breit SN et al., Growth Factors 29:187-195 (2011).
18. Johnen H et al., Cardiovasc Pathol 21:499-505 (2012).
19. Kempf T et al., Circ Res 98:351-360 (2006).
20. Wang X et al., Aging (Albany NY) 6:690-704 (2014).
21. Johnen H et al., Nat Med 13:1333-1340 (2007).
22. Breit SN, International Patent Application No PCT/AU2005/000525 (WO 2005/099746).
23. Vigano ALL et al., Cancer Res 73 Suppl1, Abstract nr 4650 (2103).
24. Lu ZH et al., Asian Pac J Cancer Prev 15:6047-6052 (2014).
25. Kempf T et al., J Am Coll Cardiol 50:1054-1060 (2007).
26. Macia L et al., PLoS One 7, e34868 (2012).
27. Chrysovergis K et al., Int J Obes (Lond) 38(12):1555-1564 (2014).
28. Hong JH et al., Diabetes Metαb J 38:472-479 (2014).
29. Tsai VW et al., PLoS One 8, e55174 (2013).
30. Mullican SE et al., Nat Med 23(10):1150-1157 (2017).
31. Vayghan HJ et al., IRJABS 4(10):3099-3103 (2013).
32. Jacquet D et al., Int J Obesity 25:491-495 (2001).
33. Obesity: preventing and managing the global epidemic. Report of a WHO consultation, World Health Organ Tech Rep Ser 894:i-xii, 1-253 (2000).
34. Bluher S et al., J Invest Med 57(7):784-788 (2009).
35. Stamatiadis DN et al., Clin Endocrinol 60(4):434-441 (2004).
36. Gonzalez LC et al., Neuroendocrinology 70(3):213-220 (1999).
37. likuni N et al., Curr Immunol Rev 4(2): 70-79 (2008).
38. Tesitore L et al., Int J Mol Med 5(4):421-426 (2000).
39. KempfT et al., Eur J Endocrinol 167:671-678 (2012).
40. Carstensen M et al., Eur J Endocrinol 162:913-917 (2010).
41. Dostalova I et al., Eur J Endocrinol 161:397-404 (2009).
42. Herder C et al., Diabetes Obes Metab 15 Suppl 3, 39-50 (2013).
43. Patricia G et al., US Patent No 6,777,388.
44. Pluckthun A., Bio/Technology 9:545-551 (1991).
45. Hudson PJ and C Souriau., Nat Med 9(1): 129-134 (2003).
46. Lonberg N., Curr Opin Immunol 20:450-459 (2008).
47. Kashmiri SVS et al. Methods 36(1): 25-34 (2005).
48. Marvin JS and HB Lowman. Phage Display in Biotechnology and Drug Discovery (2015).
49. Bowers PM et al., J Biol Chem 288(11):7688-7696 (2013).
50. Nejati-Koshki K et al., Asian Pac J Cancer Prev 14(11):6595-6599 (2014).
51. Zhang F et al., Nature 387:206-209 (1997).
52. Kovalsky I et al., Diabetes Obes Metab 12:393-402 (2010).
53. Zabeau L et al., Biol Chem 395(5):499-514 (2014).
54. Iversen PO et al., Blood 100:4123-4128 (2002).
55. De Rosa V et al., J Clin Invest 116:447-455 (2006).
56. Amirah E-E A et al., Expert Opin Drug Deliv 12(12):1923-1941 (2015).
57. Bender TS et al., Nezrroscience 303:569-576 (2015).
58. Hanson LR et al., Drug Deliv 19(3):149-154 (2012).
59. Ashima RS., J Clin Invest 118(7):2380-2383 (2008).
60. Considine RV et al., New Engl J Med 334:292-295 (1996).
61. Al Maskari MY and AA Alnaqdy., Sultan Qaboos Univ Med J 6(2):27-31 (2006).
62. Vassilev LT et al., Science 303(5659):844-848 (2004).
63. Baek SJ et al., Molec Pharm 67(2):356-364 (2005).
64. Baek, SJ et al., Mol Pharmacol 59:901-908 (2001).
65. Brown, DA et al., Cancer Epidemiol Biomarkers Prev 21 :337-346 (2012).
66. Matheny M et al., J Endocrinol 222(1):27-41 (2014).
67. Ioffe E et al., Proc Natl Acad Sci USA 95:11852-11857 (1998).
68. Maffei M et al., Nat Med 1(11):1155-1161(1995).
69. Yang L et al., Nat Med 23(10):1158-1166 (2017).
70. Emmerson PJ et al., Nat Med 23(10):1215-1219 (2017).
71. Tsai VW et al., Int J Obes (Lond) doi: 10.1038/ijo.2017.258 (2017).
72. Li Z et al., J Neurochem 95:361-376 (2005).
73. Tsai VW et al., Int J Obes (Lond) doi: 10.1038/ijo.2017.258 (2017).
74. Shpilman M et al., J Biol Chem 286:4429-4442 (2011).

## Claims

1. A composition comprising:
(i) a first agent comprising MIC-1 and/or an active MIC-1 fragment; and
(ii) a second agent comprising leptin and/or an active leptin fragment; and
optionally containing a pharmacologically acceptable carrier and/or excipient.

2. The composition according to claim 1, wherein the first agent comprises recombinant MIC-1 or synthetic MIC-1 optionally conjugated to a conjugate partner.

3. The composition according to claim 1, wherein the first agent comprises an active MIC-1 fragment optionally conjugated to a conjugate partner.

4. The composition according to any one of claims 1 to 3, wherein the second agent comprises recombinant leptin or synthetic leptin optionally conjugated to a conjugate partner.

5. The composition of any one of claims 1 to 4, wherein the second agent comprises an active leptin fragment optionally conjugated to a conjugate partner.

6. A composition according to any one of claims 1 to 5 for use in a method of controlling body weight and/or appetite, wherein said composition is administered to a subject selected from a subject that is overweight or obese, a subject with diet-induced obesity (DIO), a subject suffering from Type 2 diabetes (T2D), a subject suffering from non-alcoholic steatohepatitis (NASH), and a subject showing insulin resistance.

7. The composition for the use according to claim 6, wherein said composition is for use in a method of achieving weight loss.

8. A composition according to any one of claims 1 to 5 for use in a method for the treatment or prevention of Type 2 diabetes (T2D) and/or one or more complications of T2D, obesity or being overweight.

9. A composition for use according to claim 6, wherein said subject is an obese subject that has been determined to have a serum level of leptin of less than 30 ng/ml for an adult woman or less than 20 ng/ml for an adult man after overnight fasting.

10. A kit comprising first and second containers, wherein the first container contains a first agent comprising MIC-1 and/or an active MIC-1 fragment and the second container contains a second agent comprising leptin and/or an active leptin fragment; optionally packaged with instructions for the use of any one of claims 6 to 9.

## Patentansprüche

1. Zusammensetzung, die Folgendes umfasst:
(i) ein erstes Mittel, umfassend MIC-1 und/oder ein aktives MIC-1-Fragment; und
(ii) ein zweites Mittel, umfassend Leptin und/oder ein aktives Leptinfragment; und optional enthaltend einen pharmakologisch akzeptablen Träger und/oder Exzipienten.

2. Zusammensetzung nach Anspruch 1, wobei das erste Mittel rekombinantes MIC-1 oder synthetisches MIC-1, optional konjugiert an einen Konjugatpartner, umfasst.

3. Zusammensetzung nach Anspruch 1, wobei das erste Mittel ein aktives MIC-1-Fragment, optional konjugiert an einen Konjugatpartner, umfasst.

4. Zusammensetzung nach einem der Ansprüche 1 bis 3, wobei das zweite Mittel rekombinantes Leptin oder synthetisches Leptin, optional konjugiert an einen Konjugatpartner, umfasst.

5. Zusammensetzung nach einem der Ansprüche 1 bis 4, wobei das zweite Mittel ein aktives Leptinfragment, optional konjugiert an einen Konjugatpartner, umfasst.

6. Zusammensetzung nach einem der Ansprüche 1 bis 5 zur Verwendung in einem Verfahren zum Eindämmen des Körpergewichts und/oder des Appetits, wobei die Zusammensetzung einem Subjekt verabreicht wird, ausgewählt aus einem Subjekt, das übergewichtig oder fettleibig ist, einem Subjekt mit ernährungsbedingter Fettleibigkeit (DIO), einem Subjekt, das an Typ-2-Diabetes (T2D) leidet, einem Subjekt, das an nichtalkoholischer Steatohepatitis (NASH) leidet, und einem Subjekt, das Insulinresistenz aufweist.

7. Zusammensetzung zur Verwendung nach Anspruch 6, wobei die Zusammensetzung zur Verwendung in einem Verfahren zur Herbeiführung einer Gewichtsabnahme dient.

8. Zusammensetzung nach einem der Ansprüche 1 bis 5 zur Verwendung in einem Verfahren zur Behandlung oder Prävention von Typ-2-Diabetes (T2D) und/oder einer oder mehrerer Komplikationen von T2D, Fettleibigkeit oder Übergewicht.

9. Zusammensetzung zur Verwendung nach Anspruch 6, wobei das Subjekt ein fettleibiges Subjekt ist, bei dem bestimmt wurde, dass es einen Serumspiegel an Leptin von weniger als 30 ng/ml für eine adulte Frau oder weniger als 20 ng/ml für einen adulten Mann nach Fasten über Nacht aufweist.

10. Kit, umfassend erste und zweite Behälter, wobei der erste Behälter ein erstes Mittel enthält, das MIC-1 und/oder ein aktives MIC-1-Fragment umfasst, und der zweite Behälter ein zweites Mittel enthält, das Leptin und/oder ein aktives Leptinfragment umfasst; optional verpackt mit Anweisungen für die Verwendung nach einem der Ansprüche 6 bis 9.

## Revendications

1. Composition comprenant :
(i) un premier agent comprenant MIC-1 et/ou un fragment actif de MIC-1 ; et
(ii) un second agent comprenant de la leptine et/ou un fragment actif de leptine ; et contenant éventuellement un support et/ou un excipient pharmacologiquement acceptable.

2. Composition selon la revendication 1, dans laquelle le premier agent comprend MIC-1 recombinant ou MIC-1 synthétique éventuellement conjugué à un partenaire conjugué.

3. Composition selon la revendication 1, dans laquelle le premier agent comprend un fragment actif de MIC-1 éventuellement conjugué à un partenaire conjugué.

4. Composition selon l'une quelconque des revendications 1 à 3, dans laquelle le second agent comprend de la leptine recombinante ou de la leptine synthétique éventuellement conjuguée à un partenaire conjugué.

5. Composition de l'une quelconque des revendications 1 à 4, dans laquelle le second agent comprend un fragment actif de leptine éventuellement conjugué à un partenaire conjugué.

6. Composition selon l'une quelconque des revendications 1 à 5 pour une utilisation dans un procédé de régulation du poids corporel et/ou de l'appétit, dans laquelle ladite composition est administrée à un sujet sélectionné parmi un sujet en surpoids ou obèse, un sujet souffrant d'obésité induite par l'alimentation (DIO), un sujet souffrant de diabète de type 2 (T2D), un sujet souffrant de stéatohépatite non alcoolique (NASH) et un sujet présentant une insulinorésistance.

7. Composition pour l'utilisation selon la revendication 6, dans laquelle ladite composition est destinée à être utilisée dans un procédé permettant d'obtenir une perte de poids.

8. Composition selon l'une quelconque des revendications 1 à 5 pour une utilisation dans un procédé de traitement ou de prévention du diabète de type 2 (T2D) et/ou d'une ou plusieurs complications du T2D, de l'obésité ou du fait d'être en surpoids.

9. Composition pour une utilisation selon la revendication 6, dans laquelle ledit sujet est un sujet obèse dont le taux sérique de leptine a été déterminé comme étant inférieur à 30 ng/ml pour une femme adulte ou inférieur à 20 ng/ml pour un homme adulte après un jeûne d'une nuit.

10. Kit comprenant un premier et un second récipient, dans lequel le premier récipient contient un premier agent comprenant MIC-1 et/ou un fragment actif de MIC-1 et le second récipient contient un second agent comprenant de la leptine et/ou un fragment actif de leptine ; éventuellement emballé avec des instructions pour l'utilisation selon l'une quelconque des revendications 6 à 9.
